# EUROPÄISCHE PATENTSCHRIFT

(11) **EP 3 704 273 B1**
(45) Veröffentlichungstag und Bekanntmachung des Hinweises auf die Patenterteilung: **21.05.2025**
(21) Anmeldenummer: 18804525.6
(22) Anmeldetag: 04.11.2018
(51) Int. Cl.: C12Q 1/6886

(54) **VERFAHREN ZUR BESTIMMUNG DES ANSPRECHENS EINER MALIGNEN ERKRANKUNG AUF EINE IMMUNTHERAPIE**
METHOD OF PREDICTING RESPONSE OF A MALIGNANT DISEASE TO IMMUNTHERAPY
PROCEDE DE PREDICTION DE LA REPONSE D'UNE MALADIE MALIGNE A UNE IMMUNOTHERAPIE

(30) Priorität: 05.11.2017 DE 102017125780
(43) Veröffentlichungstag der Anmeldung: 09.09.2020
(73) Patentinhaber: Dietrich, Dimo, 10437 Berlin (DE)
(72) Erfinder: Dietrich, Dimo, 10437 Berlin (DE)
(74) Vertreter: Andresen, Heiko
(86) Internationale Anmeldenummer: PCT/EP2018/080073
(87) Internationale Veröffentlichungsnummer: WO 2019/086642

(56) Entgegenhaltungen:
- DE-B3- 102016 005 947
- DIANE GOLTZ ET AL: "CTLA4 methylation predicts response to anti-PD-1 and anti-CTLA-4 immunotherapy in melanoma patients", JCI INSIGHT, vol. 3, no. 13, 12 July 2018 (2018-07-12), XP055543596, DOI: 10.1172/jci.insight.96793
- GORAN MICEVIC ET AL: "Aberrant DNA methylation in melanoma: biomarker and therapeutic opportunities", CLINICAL EPIGENETICS, BIOMED CENTRAL LTD, GB, vol. 9, no. 1, 4 April 2017 (2017-04-04), pages 1 - 15, XP021243725, ISSN: 1868-7075, DOI: 10.1186/S13148-017-0332-8
- DAMIAN T RIEKE ET AL: "Methylation of RAD51B, XRCC3 and other homologous recombination genes is associated with expression of immune checkpoints and an inflammatory signature in squamous cell carcinoma of the head and neck, lung and cervix", 15 November 2016 (2016-11-15), XP055543508, Retrieved from the Internet <URL:https://doi.org/10.18632/oncotarget.12211> [retrieved on 20190117]
- GOLTZ: "Prognostic signifi cance and predictive value to anti-PD1 treatment of promoter methylation of PDCD1 (PD-1), CD274 (PD-L1) and PDCD1LG2 (PD-L2) for melanoma patients", 14 October 2017 (2017-10-14), XP055543873, Retrieved from the Internet <URL:https://api.wiley.com/onlinelibrary/tdm/v1/articles/10.1111%2Fddg.13299> [retrieved on 20190117]

## Beschreibung

### Verweis auf frühere Anmeldungen

Diese Patentanmeldung beansprucht die Priorität der deutschen Patentanmeldung DE 10 2017 125 780.2, deren Offenbarungsgehalt hiermit durch Rückbezug aufgenommen wird.

### Sequenzprotokoll

Die Anmeldung beinhaltet ein elektronisches Sequenzprotokoll im txt-Format nach WIPO ST.25 Standard mit 78 Sequenzen als Teil der Beschreibung.

### Gebiet der Erfindung

Die Erfindung betrifft unter anderem prädiktive molekulardiagnostische Verfahren und prädiktive Biomarker sowie deren Verwendungen, mit denen in der onkologischen Medizin ein Ansprechen einer malignen Erkrankung auf eine Immuntherapie vorhergesagt wird. Ferner betrifft die Erfindung ein Kit zur Durchführung der angegebenen Verfahren.

### Hintergrund der Erfindung

Die personalisierte Medizin beruht auf der Entwicklung von Therapien, die auf Patienten mit spezifischen Erkrankungen abgestimmt sind. Einen vielversprechenden Ansatz in der Onkologie stellen immuntherapeutische Behandlungen mit sogenannten Immun-Checkpoint-Inhibitoren dar, weil sie auch bei fortgeschrittenen Tumorerkrankungen herausragende Ergebnisse zeigen können. Problematisch ist allerdings, dass selbst Patienten mit identischem Krankheitsbild auf die gleiche Therapie oder den gleichen Wirkstoff unterschiedlich ansprechen können.

Prädiktive molekulardiagnostische Verfahren ("Companion Diagnostics" und "Complementary Diagnostics") und prädiktive Biomarker ("Companion Biomarkers" und "Complementary Biomarkers") können bei der Vorhersage helfen, wie gut ein Patient auf eine bestimmte Behandlung oder einen bestimmten Wirkstoff ansprechen wird. Aus DE 10 2016 005 947 B3 ist zum Beispiel bekannt, dass eine DNA-Methylierungsanalyse des *PD-1* Gens von Zellen einer malignen Erkrankung oder von mit den Zellen der malignen Erkrankung wechselwirkenden T-Lymphozyten eine Vorhersage ermöglicht, ob der Patient auf eine Immuntherapie mit Wirkstoffen ansprechen wird, welche den PD-1 Rezeptor oder dessen Liganden inhibieren. Micevic et al. (Clinical Epigenetics 2017, 9:34) befasst sich mit aberranter DNA-Methylierung im Melanom. In Rieke et al. (Oncotarget 2016, 7:75379-75393) wird eine Korrelation zwischen der Methylierung verschiedener DNA-Reparaturegene und der Expression von CD274 und CTLA4 untersucht. Goltz et al. (Journal of the German Society of Dermatology 2017, 15, Suppl. 3:1-88) beschreibt einen Zusammenhang zwischen der Promotormethylierung von CD274 und PDCD1LG2 und dem Ansprechen auf eine Immuntherapie, die sich gegen die PD-1/PD-L1-Achse richtet.

Um das Nutzen-Risiko-Verhältnis solcher Immuntherapien weiter zu verbessern, ist es erforderlich, die klinische Entscheidungsfindung durch eine noch genauere Subtypisierung von Patientengruppen zu unterstützen. Es werden weitere beziehungsweise komplementäre prädiktive Verfahren und Biomarker benötigt, um die Auswahl von Patienten angesichts vielfältiger aufkommender Behandlungsoptionen noch sicherer zu leiten.

### Zusammenfassung der Erfindung

Vor diesem Hintergrund stellt die vorliegende Erfindung in einem ersten Aspekt ein Verfahren zur Vorhersage eines Ansprechens einer malignen Erkrankung auf eine Immuntherapie, die dazu ausgelegt ist, einen PD-1-Immun-Checkpoint-Signalweg zu inhibieren, bereit. Das Verfahren kennzeichnet, dass zumindest ein Teil eines immunregulatorischen Gens ausgewählt aus *CTLA4, CD86, CD28, CD80* und/oder *ICOS* von Zellen der malignen Erkrankung und/oder von mit den Zellen der malignen Erkrankung wechselwirkenden Immunzellen einer DNA-Methylierungsanalyse unterzogen wird. Anschließend wird anhand des Ergebnisses der DNA-Methylierungsanalyse, das heißt anhand von Anwesenheit, Abwesenheit und/oder Ausmaß einer DNA-Methylierung des immunregulatorischen Gens, das Ansprechen der malignen Erkrankung auf die Immuntherapie vorhergesagt. Die Immuntherapie umfasst einen Wirkstoff, welcher den PD-1-Immun-Checkpoint-Signalweg durch eine Bindung des Wirkstoffs an PD-1, PD-L1 und/oder PD-L2 inhibiert, wobei der Wirkstoff einen anti-PD-1 Antikörper, einen anti-PD-L1 Antikörper und/oder einen anti-PD-L2 Antikörper umfasst.

Der zweite Aspekt der Erfindung betrifft ein Verfahren zur Auswahl eines Patienten, welcher an einer malignen Erkrankung leidet, für eine auf die Inhibierung eines PD-1-Immun-Checkpoint-Signalwegs ausgelegte Immuntherapie. Das Verfahren umfasst die Schritte A) Bereitstellen von Zellen der malignen Erkrankung und/oder von mit den Zellen der malignen Erkrankung wechselwirkenden Immunzellen des Patienten, B) Durchführen einer DNA-Methylierungsanalyse zumindest eines Teils eines immunregulatorischen Gens ausgewählt aus *CTLA4, CD86, CD28, CD80* und/oder *ICOS* von den Zellen der malignen Erkrankung und/oder von mit den Zellen der malignen Erkrankung wechselwirkenden Immunzellen aus A), sowie C) Auswählen des Patienten für die Immuntherapie anhand von Anwesenheit, Abwesenheit und/oder Ausmaß der in B) untersuchten beziehungsweise ermittelten DNA-Methylierung des immunregulatorischen Gens. Die Immuntherapie umfasst einen Wirkstoff, welcher den PD-1-Immun-Checkpoint-Signalweg durch eine Bindung des Wirkstoffs an PD-1, PD-L1 und/oder PD-L2 inhibiert, wobei der Wirkstoff einen anti-PD-1 Antikörper, einen anti-PD-L1 Antikörper und/oder einen anti-PD-L2 Antikörper umfasst.

Der dritte Aspekt der Erfindung betrifft die Verwendung einer DNA-Methylierungsanalyse zumindest eines Teils eines immunregulatorischen Gens ausgewählt aus *CTLA4, CD86, CD28, CD80* und/oder *ICOS* von Zellen einer malignen Erkrankung und/oder von mit den Zellen der malignen Erkrankung wechselwirkenden Immunzellen zur Vorhersage eines Ansprechens der malignen Erkrankung auf eine Immuntherapie, zur individualisierten Auswahl einer Immuntherapie für die malignen Erkrankung und/oder zur Auswahl eines Patienten, welcher an der malignen Erkrankung leidet, für eine Immuntherapie, wobei die Immuntherapie wiederum dazu ausgelegt ist, einen PD-1-Immun-Checkpoint-Signalweg zu inhibieren. Die Immuntherapie umfasst einen Wirkstoff, welcher den PD-1-Immun-Checkpoint-Signalweg durch eine Bindung des Wirkstoffs an PD-1, PD-L1 und/oder PD-L2 inhibiert, wobei der Wirkstoff einen anti-PD-1 Antikörper, einen anti-PD-L1 Antikörper und/oder einen anti-PD-L2 Antikörper umfasst.

Der vierte Aspekt der Erfindung betrifft die Verwendung von Anwesenheit, Abwesenheit oder Ausmaß einer DNA-Methylierung von zumindest einem Teil eines immunregulatorischen Gens ausgewählt aus *CTLA4, CD86, CD28, CD80* und/oder *ICOS* von Zellen einer malignen Erkrankung und/oder von mit den Zellen der malignen Erkrankung wechselwirkenden Immunzellen als Biomarker zur Vorhersage eines Ansprechens der malignen Erkrankung auf eine Immuntherapie, zur individualisierten Auswahl einer Immuntherapie für die malignen Erkrankung und/oder zur Auswahl eines Patienten, welcher an der malignen Erkrankung leidet, für eine Immuntherapie, wobei die Immuntherapie dazu ausgelegt ist, einen PD-1-Immun-Checkpoint-Signalweg zu inhibieren. Die Immuntherapie umfasst einen Wirkstoff, welcher den PD-1-Immun-Checkpoint-Signalweg durch eine Bindung des Wirkstoffs an PD-1, PD-L1 und/oder PD-L2 inhibiert, wobei der Wirkstoff einen anti-PD-1 Antikörper, einen anti-PD-L1 Antikörper und/oder einen anti-PD-L2 Antikörper umfasst.

Gemäß einem fünften Aspekt betrifft die Erfindung die Verwendung eines Kits zur Durchführung eines der vorgenannten Verfahren. Das Kit umfasst Reagenzien für die DNA-Methylierungsanalyse zumindest eines Teils eines immunregulatorischen Gens ausgewählt aus *CTLA4, CD86, CD28, CD80* und/oder *ICOS* von Zellen einer malignen Erkrankung und/oder von mit den Zellen der malignen Erkrankung wechselwirkenden Immunzellen, um Anwesenheit, Abwesenheit und/oder Ausmaß der DNA-Methylierung des immunregulatorischen Gens zu bestimmen.

### Definitionen und allgemeine Erläuterungen

In dieser Beschreibung werden verschiedene Dokumente zitiert, um einen allgemeinen technischen Hintergrund in Bezug auf die vorliegende Erfindung zu vermitteln. Auf die Offenbarung und Lehre dieser Dokumente wird in Ergänzung nachfolgender Beschreibung vollinhaltlich Bezug genommen, um Wiederholungen zu vermeiden.

Die nachfolgenden Definitionen und allgemeinen Erläuterungen sollen den fachkundigen Leser beim Verständnis, in der Auslegung und bei der Ausübung der vorliegenden Erfindung anleiten und unterstützen. Vorbehaltlich anderer Angaben sollen alle technischen und wissenschaftlichen Begriffe diejenige Bedeutung haben, welche dem üblichen Begriffsverständnis eines Durchschnittsfachmanns auf dem Gebiet der vorliegenden Erfindung entspricht.

Die verschiedenen Aspekte und Varianten der Erfindung involvieren Techniken und Methoden aus der molekularbiologischen Routinepraxis. Insbesondere gehört die DNA-Methylierungsanalyse zur Bestimmung der Methylierung eines CpG-Dinukleotids zu den Fachkenntnissen eines Molekularbiologen oder -genetikers. Zweckdienliche Laborhandbücher für diese Techniken und Methoden stehen dem Fachmann ohne Weiteres zur Verfügung, beispielsweise "Molecular Cloning, A Laboratory Manual" von M.R. Green und J. Sambrook, 4th Ed., 2012, Cold Spring Harbor Laboratory Press.

So, wie sie hier verwendet werden, schließen unbestimmte Artikel wie "ein" oder "eine" die Möglichkeit mit ein, dass auch zwei oder mehrere dieser Merkmale vorhanden sein können.

So, wie der Begriff hier verwendet wird, umfassen "maligne Erkrankungen" oder auch "bösartige Erkrankungen" solche Erkrankungen, die durch einen Krankheitsverlauf gekennzeichnet sind, der fortschreitend zerstörerisch ist und möglicherweise auch zum Tod des Patienten führen kann. Maligne Erkrankungen umfassen maligne Gewebeneubildungen, zum Beispiel Neoplasien oder Tumoren, wobei die Malignität durch unkontrolliertes, raumforderndes, verdrängendes, infiltratives und/oder invasives Wachstum gekennzeichnet sein kann. Maligne Tumoren sind in der Regel in der Lage, Tochtergeschwülste (Metastasen) zu bilden. Maligne Tumoren sind zum Beispiel Karzinome, Sarkome, Melanome, Glioblastome, Blastome, Seminome und Teratome. Maligne Erkrankungen umfassen auch hämatologische maligne Erkrankungen, das heißt bösartige Erkrankungen, die das Blutsystem oder das blutbildende System betreffen, beispielsweise Leukämien, Lymphome, myeloproliferative Erkrankungen und myelodysplastische Syndrome. Leukämien umfassen eine Gruppe von malignen Erkrankungen, bei denen unreife hämatopoetische Zellen sich bösartig verändert haben, sich übermäßig stark vermehren und zu einer Anhäufung von Zellen im peripheren Blut führen. Lymphome umfassen Erkrankungen, bei denen Zellen des lymphatischen Systems entartet sind. Myeloproliferative Erkrankungen umfassen eine Gruppe von Erkrankungen, bei denen eine oder mehrere blutbildende Zellreihen stark vermehrt auftreten. Myelodysplastische Syndromen umfassen eine klonale Expansion von Vorläuferzellen aller blutbildenden Zellreihen, wobei eine chronisch verlaufende Differenzierungsstörung der blutbildenden Stammzellen zugrunde liegt.

"Immuntherapie" beziehungsweise "immuntherapeutische Behandlung" wird hier als Sammelbegriff für alle Behandlungsansätze verwendet, mit denen die Aktivität des Immunsystems beeinflusst werden soll. Eine Immuntherapie kann darauf gerichtet sein, die Wirkung des Immunsystems zu stärken oder sie abzuschwächen. In bestimmten Varianten der Erfindung umfasst eine Immuntherapie eine Behandlung mit Wirkstoffen, um die organismuseigene Immunreaktion gegen die maligne Erkrankung zu verstärken. Solche Wirkstoffe umfassen Immun-Checkpoint-Inhibitoren wie zum Beispiel monoklonale Antikörper, welche spezifisch an Immun-Checkpoints binden und auf diese Weise deren (insbesondere antiinflammatorische) Signalgebung verhindern. Eine andere Möglichkeit sind Wirkstoffe, welche bereits die Expression der Immun-Checkpoints unterbinden, beispielsweise durch RNA-Interferenz.

Das "Ansprechen" auf eine Immuntherapie kann nach den Kriterien zur Bewertung immun-bedingten Ansprechens solider Tumoren (immune-related Response Evaluation Criteria In Solid Tumors - irRECIST) beurteilt werden (Nishino et al., J Immunother Cancer, 2014, 2:17; Wolchok et al., Clin Cancer Res. 2009, 15:7412-20). Insbesondere kann ein Ansprechen auf eine Immuntherapie durch komplette oder partielle Remission, durch einen unveränderten (stabilen) Zustand sowie durch eine Verzögerung eines oder mehrerer der folgenden Ereignisse gekennzeichnet sein: Eintritt des Todes, Auftreten eines Rezidivs, Auftreten von Lymphknotenmetastasen, Auftretens von Fernmetastasen, Progression der malignen Erkrankung. Ein Ansprechen auf eine Immuntherapie kann auch durch verzögerten Anstieg oder Rückgang eines sonstigen Parameters, welcher spezifisch für die maligne Erkrankung ist, gekennzeichnet sein. Beispielsweise kann ein Rückgang oder ein verzögerter Anstieg des Prostata-spezifischen Antigens (PSA) im Blut das Ansprechen eines Prostatakarzinoms auf eine Immuntherapie kennzeichnen. Das Ausbleiben des Ansprechens kann dadurch gekennzeichnet sein, dass das Ausmaß der malignen Erkrankung zunehmend, oder beschleunigt zunehmend ist. Als Vergleich kann jeweils das Ausmaß der malignen Erkrankung vor Anwendung der Therapie dienen. Das Ausmaß der Erkrankung kann durch die Anzahl der malignen Zellen, die Anzahl der Metastasen beziehungsweise die Größe des malignen Tumors charakterisiert sein.

So, wie der Begriff hier verwendet wird, bedeutet "einen PD-1-Immun-Checkpoint-Signalweg inhibieren" oder "einen CTLA4-Immun-Checkpoint-Signalweg inhibieren" ein Verlangsamen, Hemmen oder Verhindern einer oder mehrerer Reaktionen chemischer, biologischer und/oder physikalischer Natur, welche durch eine Wechselwirkung des PD-1-Immun-Checkpoints mit seinen Liganden PD-L1 und/oder PD-L2 beziehungsweise des CTLA4-Immun-Checkpoints mit seinen Liganden CD86 und/oder CD80 vermittelt werden.

Unter "DNA-Methylierung" versteht man die biochemische oder chemische Kopplung von Methylgruppen an bestimmte Nukleotide der DNA. Im Rahmen dieser Erfindung bezieht sich "DNA-Methylierung" auf das Vorliegen einer Methylgruppe am fünften Kohlenstoffatom eines Cytosins (5-Methylcytosin), welches sich in einem CpG-Dinukleotidkontext befindet. Ein "CpG-Dinukleotid" ist ein DNA-Motiv, welches in allgemein gültiger Leserichtung von 5' nach 3' die Nukleosidfolge Cytidin-Phosphat-Guanosin aufweist. Guanosin besteht aus der Nukleinbase Guanin und dem Zucker β-D-Ribose. Cytidin besteht aus der Nukleinbase Cytosin und dem Zucker β-D-Ribose.

Eine "DNA-Methylierungsanalyse" im Sinne der vorliegenden Erfindung umfasst demnach die Bestimmung einer solchen DNA-Methylierung eines CpG-Dinukleotids oder mehrerer CpG-Dinukleotide aus einem bestimmten Sequenzkontext. In verschiedenen Varianten der Erfindung wird unter "DNA-Methylierungsanalyse" die Ermittlung verstanden, ob das Cytosin in dem oder den CpG-Dinukleotid(en) methyliert vorliegt. Sofern eine Vielzahl von Genomkopien untersucht wird, kann mit der DNA-Methylierungsanalyse auch das Ausmaß der DNA-Methylierung bestimmt werden. Dazu wird beispielsweise ermittelt, wie häufig das Cytosin durchschnittlich in dem oder den CpG-Dinukleotid(en) in der Vielzahl der Genomkopien methyliert vorliegt, nachfolgend auch als "Methylierungsgrad" bezeichnet.

Eine "Komethylierung" bedeutet eine Korrelation der DNA-Methylierung zwischen zwei oder mehreren CpG-Dinukleotiden. Solche korrelierenden Methylierungen treten regelmäßig bei CpG-Dinukleotiden auf, die im Genom benachbart sind und/oder die sich innerhalb benachbarter, strukturell und/oder funktionell zusammenhängender Gene befinden, welche zusammen exprimiert werden können. Aus der DNA-Methylierung des einen Gens lässt sich daher auf die DNA-Methylierung des anderen, komethylierten Gens schließen.

Als "Gen" im Sinne der Erfindung wird ein Abschnitt auf der DNA bezeichnet, der regulatorische, transkribierte und/oder funktionelle Sequenzregionen umfasst und somit die Grundinformationen zur Herstellung einer biologisch aktiven RNA enthält. Insbesondere umfasst ein Gen auch diejenigen Elemente, wie beispielsweise Promotor, Transkriptionsfaktor-Bindestellen, CpG-Inseln, offenes Chromatin, Enhancer und Silencer, CTCF-Bindestellen, welche bei der Transkription des Gens eine regulatorische Funktion erfüllen.

Die Nomenklatur für die Bezeichnung von Genen und deren Nukleotiden erfolgt entsprechend der Empfehlung des "Human Genome Organisation Gene Nomenclature Committee" (HGNC) mit Stand vom 31. Oktober 2017. Ein Genstamm wird beispielsweise mit kursiven lateinischen Großbuchstaben bezeichnet (z.B. *CTLA4, CD86).* Die hier beschriebenen Gene sind über die "GenBank" des National Institute of Health, USA, mit Stand vom 31. Oktober 2017 öffentlich verfügbar (Benson D.A. et al., Nucleic Acids Research, 2013, 41, D36-42).

Wenn in der nachfolgenden Beschreibung Bezug auf bestimmte DNA-Sequenzen (SEQ ID NOs) genommen wird, schließt dies immer auch Sequenzvarianten mit mindestens 95%, mindestens 96%, mindestens 97%, mindestens 98% oder mindestens 99% Sequenzidentität mit der genannten DNA-Sequenz mit ein. Die Sequenzidentität von zwei Nukleinsäuresequenzen kann zum Beispiel mit dem Algorithmus ClustalW bestimmt werden (Thompson et al., Nucleic Acids Research, 1994, 22, 4673-4680).

Im Sinne der vorliegenden Erfindung umfasst "mit den Zellen der malignen Erkrankung wechselwirkende Immunzellen" solche Immunzellen, die mit Zellen der malignen Erkrankung zum Beispiel über eine Liganden-Rezeptor-Bindung spezifisch in Kontakt stehen beziehungsweise in Kontakt treten können. Der Ligand kann sich auf der Oberfläche von Zellen der malignen Erkrankung befinden, zum Beispiel ein MHC:Peptid-Komplex beziehungsweise ein MHC I:Antigen-Komplex oder ein Epitop. Der Rezeptor kann sich auf der Oberfläche der Immunzellen befinden, zum Beispiel ein T-Zell-Rezeptor oder B-Zell-Rezeptor. Alternativ kann sich der Ligand auf der Oberfläche der Immunzellen und der Rezeptor auf der Oberfläche von Zellen der malignen Erkrankung befinden. "Mit den Zellen der malignen Erkrankung wechselwirkende Immunzellen" umfasst deshalb beispielsweise auch T-Lymphozyten und/oder B-Lymphozyten, die durch den Kontakt mit einem Antigen oder antigenpräsentierenden Zellen in die Lage versetzt (aktiviert) wurden, spezifisch mit Zellen der malignen Erkrankung über eine der vorgenannten Liganden-Rezeptor-Bindungen zu wechselwirken, ohne selbst zuvor mit den entsprechenden Zellen der malignen Erkrankung in Kontakt gekommen zu sein. Eine antigenpräsentierende Zelle kann beispielsweise eine dendritische Zelle, eine Makrophage oder ein B-Lymphozyt sein. Die Aktivierung erfolgt beispielsweise über eine Liganden-Rezeptor-Bindung zwischen T-Lymphozyten und einer antigenpräsentierenden Zelle, welche ein aus einer Zelle der malignen Erkrankung stammendes Antigen präsentiert. Der Ligand, zum Beispiel ein MHC II:Antigen-Komplex, befindet sich auf der Oberfläche der antigenpräsentierenden Zellen und der T-Zell-Rezeptor befindet sich auf der Oberfläche des T-Lymphozyten. Eine weitere Form der Wechselwirkung zwischen T-Lymphozyten und Zellen der malignen Erkrankung im Sinne der Erfindung beinhaltet, dass von den Zellen der malignen Erkrankung gebildetes Adenosin durch einen Rezeptor auf der Oberfläche der T-Lymphozyten gebunden wird. Es ist auch möglich, dass das Adenosin durch T-Lymphozyten gebildet wird und sich der Rezeptor auf der Oberfläche von Zellen der malignen Erkrankung befindet. Im Sinne der vorliegenden Erfindung umfasst "mit den Zellen der malignen Erkrankung wechselwirkende Immunzellen" ferner auch solche Immunzellen, insbesondere T-Lymphozyten und/oder B-Lymphozyten, die über Wachstumsfaktoren oder/und Zytokine mit den Zellen der malignen Erkrankung wechselwirken.

"Biomarker" sind charakteristische Indikatoren oder/und biologische Merkmale, die objektiv gemessen werden können und Rückschluss über den Status eines normalen biologischen oder eines krankhaften Prozesses in einem Organismus, beziehungsweise die Antwort eines normalen oder krankhaften Prozesses auf eine Intervention, beispielsweise eine Operation, eine Bestrahlung oder eine medikamentöse Behandlung, zulassen. Biomarker sind häufig (bio-)chemische Substanzen, wie zum Beispiel Proteine, Hormone, Metaboliten, Zucker und Nukleinsäuren, sowie Modifikationen davon.

Sowohl die vorstehende allgemeine Beschreibung als auch die nachfolgende detaillierte Beschreibung sind als Beispiel aufzufassen und sollen zur Erläuterung der beanspruchten Erfindung dienen. Weitere Vorteile und Merkmale der Erfindung sind aus der nachstehenden Beschreibung, den Zeichnungen und den Patentansprüchen ersichtlich. Auch wenn die Erfindung anhand ihrer bevorzugten Ausführungsformen erläutert wird, können viele weitere Variationen vorgenommen werden, ohne über den Umfang der vorliegenden Erfindung hinauszugehen. Daher ist es vorgesehen, dass die beiliegenden Patentansprüche Variationen und Kombinationen von Merkmalen abdecken, die im tatsächlichen Umfang der Erfindung enthalten sind, auch wenn diese nicht ausdrücklich in den Ansprüchen abgebildet sind.

### Kurze Beschreibung der Figuren

Figur 1 zeigt einen Boxplot zur Verteilung der DNA-Methylierung von *CTLA4* in malignen Melanomen bei Patienten mit progressiver Erkrankung, stabiler Erkrankung, partieller und kompletter Remission während einer Immuntherapie, die dazu ausgelegt ist den PD-1-Immun-Checkpoint-Signalweg zu inhibieren. Die DNA-Methylierung wurde vor Beginn der Immuntherapie bestimmt. Die Patienten wurden retrospektiv nach irRECIST Kriterien gruppiert.
Figur 2 zeigt eine Kaplan-Meier Analyse des Gesamtüberlebens von 50 Patienten mit malignen Melanomen während einer Immuntherapie, die dazu ausgelegt ist den PD-1-Immun-Checkpoint-Signalweg zu inhibieren. Die Patienten wurden in einer dreistufigen Bewertung anhand von *CTLA4* DNA-Methylierung gruppiert. Das untere Tertil umfasst die 17 Patienten mit der niedrigsten gemessenen DNA-Methylierung, das obere Tertil die 17 Patienten mit der höchsten gemessenen DNA-Methylierung und das mittlere Tertil die übrigen 16 Patienten.
Figur 3 zeigt eine Komethylierungs-Matrix verschiedener Genloci von *CTLA4, CD28, CD80, CD86* und *ICOS* in 419 Urothelkarzinomen der Blase.
Figur 4 zeigt eine Komethylierungs-Matrix verschiedener Genloci von *CTLA4, CD28, CD80, CD86* und *ICOS* in 797 Brustkrebstumoren.
Figur 5 zeigt eine Komethylierungs-Matrix verschiedener Genloci von *CTLA4, CD28, CD80, CD86* und *ICOS* in 530 Plattenepithelkarzinomen des Kopf- und Hals-Bereichs.
Figur 6 zeigt eine Komethylierungs-Matrix verschiedener Genloci von *CTLA4, CD28, CD80, CD86* und *ICOS* in 325 klarzelligen Nierenzellkarzinomen.
Figur 7 zeigt eine Komethylierungs-Matrix verschiedener Genloci von *CTLA4, CD28, CD80, CD86* und *ICOS* in 475 Adenokarzinomen der Lunge.
Figur 8 zeigt eine Komethylierungs-Matrix verschiedener Genloci von *CTLA4, CD28, CD80, CD86* und *ICOS* in 370 Plattenepithelkarzinomen der Lunge.
Figur 9 zeigt eine Komethylierungs-Matrix verschiedener Genloci von *CTLA4, CD28, CD80, CD86* und *ICOS* in 265 Sarkomen.
Figur 10 zeigt eine Komethylierungs-Matrix verschiedener Genloci von *CTLA4, CD28, CD80, CD86* und *ICOS* in 473 kutanen Melanome.

### Kurze Beschreibung der Sequenzen

SEQ ID NO:1 bis SEQ ID NO:78 wie jeweils unter der numerischen Kennzahl <213> beziehungsweise <223> des Sequenzprotokolls als Teil der Beschreibung angegeben.

### Beschreibung der Erfindung

Immun-Checkpoints stellen Schlüsselziele für Immuntherapien dar. Bei der Behandlung verschiedener maligner Erkrankungen hat sich eine sogenannte Immun-Checkpoint Blockade (ICB) als besonders effizient erwiesen. Dabei werden mithilfe von Immun-Checkpoint-Inhibitoren die Signalwege von immunsupprimierenden Immun-Checkpoints unterbrochen, damit das körpereigene Immunsystem die entarteten Zellen besser erkennen und bekämpfen kann. Weil aber immuntherapeutische Medikamente in der Regel nur bei bestimmten Patienten wirken und eine etwaige Wirkung oft erst nach mehreren Monaten zu beobachten ist, ist es für die klinische Praxis von größter Bedeutung, bereits im Vorfeld einer Therapie Anhaltspunkte dafür zu haben, ob ein Patient auf eine bestimmte Behandlung ansprechen wird.

Der Erfinder der vorliegenden Erfindung konnte in der DE 10 2016 005 947 B3 bereits zeigen, dass in Zellen maligner Erkrankungen beziehungsweise in mit den Zellen der malignen Erkrankung wechselwirkenden T-Lymphozyten die DNA-Methylierung zentraler immunregulatorischer Gene wie zum Beispiel das für PD-1 codierende Gen *PDCD1* unmittelbar mit der Expression der von diesen Genen codierten Immun-Checkpoints korreliert ist. Der Erfinder hat weiterhin erkannt, dass aufgrund dieses Zusammenhangs mithilfe einer DNA-Methylierungsanalyse dieser immunregulatorischen Gene eine Vorhersage über das Ansprechen eines Patienten auf eine Immuntherapie möglich ist. Demzufolge ist beispielsweise ein Ansprechen auf einen Wirkstoff, welcher den PD-1 Rezeptor inhibiert, wahrscheinlicher, wenn die DNA-Methylierungsanalyse des entsprechenden *PDCD-1* Gens von Zellen der malignen Erkrankung oder von mit den Zellen der malignen Erkrankung wechselwirkenden T-Lymphozyten anzeigt, dass der PD-1 Rezeptor zunächst von den Zellen exprimiert wird.

Im Rahmen intensiver weiterführender Forschungsarbeit und aufwändiger klinischer Studien hat der Erfinder nun aufzeigen können, dass überraschenderweise auch eine DNA-Methylierung der immunregulatorischen Gene *CTLA4, CD86, CD28, CD80* und *ICOS* mit besonders hoher Zuverlässigkeit anzeigt, ob eine maligne Erkrankung auf eine Immuntherapie anspricht, mit der ein PD-1-Immun-Checkpoint-Signalweg inhibiert wird. Diesbezüglich wird auch auf die nachfolgenden Ausführungsbeispiele verwiesen. Diese neue Erkenntnis ist aus fachlicher Sicht nicht zu erwarten gewesen, weil diese Gene für Immun-Checkpoints codieren, welche in keinem unmittelbaren Zusammenhang mit dem PD-1-Immun-Checkpoint-Signalweg stehen. Daher unterscheiden sich die neuen Erkenntnisse des Erfinders grundlegend von der Offenbarung und

Lehre der DE 10 2016 005 947 B3, welche sich darauf beschränkt, dass sich eine DNA-Methylierungsanalyse eines immunreglatorischen Gens zur Vorhersage des Ansprechens auf eine Immuntherapie eignet, die gegen den durch dasselbe immunregulatorische Gen codierten Immun-Checkpoint gerichtet ist.

Vor diesem Hintergrund betrifft der erste Aspekt der vorliegenden Erfindung ein Verfahren zur Vorhersage eines Ansprechens einer malignen Erkrankung auf eine Immuntherapie, die dazu ausgelegt ist, einen PD-1-Immun-Checkpoint-Signalweg zu inhibieren. Dabei wird zumindest ein Teil eines immunregulatorischen Gens von Zellen der malignen Erkrankung und/oder von mit den Zellen der malignen Erkrankung wechselwirkenden Immunzellen einer DNA-Methylierungsanalyse unterzogen, um das immunregulatorische Gen auf DNA-Methylierung zu untersuchen. Das immunregulatorische Gen ist hierbei aus der Gruppe bestehend aus *CTLA4, CD86, CD28, CD80, ICOS* oder beliebigen Kombinationen davon ausgewählt. Auf der Grundlage dieser DNA-Methylierungsanalyse wird anschließend anhand von Anwesenheit, Abwesenheit und/oder Ausmaß der DNA-Methylierung des immunregulatorischen Gens das Ansprechen der malignen Erkrankung auf die Immuntherapie vorhergesagt (Prädiktion).

Es zeichnet die vorliegende Erfindung besonders aus, dass sich die DNA-Methylierungsanalyse der erfindungsgemäßen immunregulatorischen Gene universell für die Vorhersage des Ansprechverhaltens verschiedenster maligner Erkrankungen beziehungsweise Tumorentitäten eignet. Diese Entdeckung des Erfinders steht im Einklang mit dem neuesten Wissenststand der U.S. Food and Drug Administration, die kürzlich erstmals eine Immuntherapie von Krebserkrankungen basierend auf einer gemeinsamen genetischen Eigenschaft der Erkrankungen zugelassen hat, anstatt wie vorher üblich die Zulassung anhand des erkrankten Organs zu definieren (Chang et al., Appl Immunohistochem Mol Morphol. 2017, Epub ahead of print). Die maligne Erkrankung kann insbesondere ein Melanom, ein Karzinom, ein Sarkom, ein Glioblastom, ein Lymphom und/oder eine Leukämie umfassen. Das Karzinom kann zum Beispiel ein Adenokarzinom, ein Plattenepithelkarzinom, ein kleinzelliges Karzinom, ein neuroendokrines Karzinom, ein Nierenzellkarzinom, ein Urothelkarzinom, ein hepatozelluläres Karzinom, ein Analkarzinom, ein Bronchialkarzinom, ein Endometriumkarzinom, ein cholangiozelluläres Karzinom, ein hepatozelluläres Karzinom, ein Hodenkarzinom, ein kolorektales Karzinom, ein Karzinom des Kopf- und Halsbereichs, ein Karzinom des Ösophagus, ein Magenkarzinom, ein Mammakarzinom, ein Nierenkarzinom, ein Ovarialkarzinom, ein Pankreaskarzinom, ein Prostatakarzinom, ein Schilddrüsenkarzinom und/oder ein Zervixkarzinom umfassen. Ein Sarkom kann beispielsweise ein Angiosarkom, ein Chondrosarkom, ein Ewing-Sarkom, ein Fibrosarkom, ein Kaposi-Sarkom, ein Liposarkom, ein Leiomyosarkom, ein malignes fibröses Histiozytom, ein neurogenes Sarkom, ein Osteosarkom oder ein Rhabdomyosarkom sein. Eine Leukämie kann beispielsweise eine akute myeloische Leukämie (AML), eine akute lymphatische Leukämie (ALL), eine chronische lymphatische Leukämie (CLL), oder eine chronische myeloische Leukämie (CML) sein. Ein Lymphom kann ein Hodgkin-Lymphom oder Non-Hodgkin-Lymphom sein. Ein Non-Hodgkin-Lymphom kann ein B-Zell-Lymphom oder ein T-Zell-Lymphom sein. Insbesondere handelt es sich bei der malignen Erkrankung um ein, gegebenenfalls metastasiertes, malignes Melanom.

Die Immuntherapie umfasst einen Wirkstoff, welcher an PD-1, PD-L1 und/oder PD-L2 bindet und durch diese Bindung den PD-1-Immun-Checkpoint-Signalweg inhibiert, indem die natürliche Wechselwirkung zwischen dem PD-1-Rezeptor und seinen Liganden unterbunden wird, wobei der Wirkstoff einen anti-PD-1 Antikörper, einen anti-PD-L1 Antikörper und/oder einen anti-PD-L2 Antikörper umfasst. Vorzugsweise handelt es sich hierbei um einen monoklonalen Antikörper. Der Wirkstoff kann zum Beispiel aus Nivolumab (BMS-936558, Handelsname: Opdivo, Hersteller: Bristol-Myers Squibb), Pembrolizumab (MK-3475, SCH900475, Handelsname: Keytruda^{®}; Hersteller: Merck/ MSD Sharp & Dohme), Pidilizumab (CT-011, MDV9300; Hersteller: CureTech Ltd., lizensiert durch Medivation), MGD013 (Macrogenics), AMP-224 (Hersteller: GlaxoSmithKline), MEDI0680 (AMP-514, Hersteller: MedImmune LLC), AUNP-12 (Hersteller: Aurigene Discovery Technologies Ltd.), BMS935559 (MDX-1105, Hersteller: Bristol-Myers Squibb), CA-170 und CA-237 (Hersteller: Curis Inc.), MPDL3280A (Hersteller: Roche), MEDI4736 (Hersteller: AstraZeneca), Avelumab (MSB0010718C, Hersteller: Pfizer) und/oder rHIgM12B7 (B7-DC cross-linking antibody rHIgM12B7, Mayo Clinic), TSR-042 (Hersteller: Tesaro), SHR-1210 (Hersteller: Jiangsu Hengrui Medicine Co., Ltd.), Sym021 (Hersteller: Symphogen A/S), REGN2810 (Hersteller: Regeneron), JNJ-63723283 (Hersteller: JoJanssen Research & Development, LLC), BGB-A317 (Hersteller: BeiGene), PDR001 (Hersteller: Novartis), JTX-4014 (Hersteller: Jounce Therapeutics), Atezolizumab (MPDL3280A, Hersteller: Genentech/Roche), Durvalumab (MEDI4736, MEDI-4736, Hersteller: Medimmune/AstraZeneca), LY3300054 (Hersteller: Lilly), KN035 (Hersteller: Suzhou Alphamab Co. Ltd.), CX-072 (Hersteller: CytomX Therapeutics) und beliebigen Kombinationen davon ausgewählt sein.

Die DNA-Methylierungsanalyse kann im Grunde genommen mit allen gängigen Methoden durchgeführt werden, welche dem Fachmann aus der einschlägigen Literatur bekannt sind. Ein geeignetes Verfahren umfasst beispielsweise die folgenden Schritte: A) Bereitstellen von DNA der Zellen der malignen Erkrankung beziehungsweise der mit den Zellen der malignen Erkrankung wechselwirkenden Immunzellen; B) Umwandeln zumindest eines Teils der in der DNA aus A) enthaltenen Cytosine in Uracil oder eine andere Base mit einem von Cytosin unterscheidbaren Basenpaarungsverhalten und/oder Molekulargewicht; C) Untersuchen der aus Schritt B) erhaltenen DNA auf eine DNA-Methylierung des immunregulatorischen Gens.

Die zu analysierende DNA aus Schritt A) kann aus verschiedenen Quellen stammen und beispielsweise Zellen der malignen Erkrankung oder infiltrierende Immunzellen, insbesondere tumorinfiltrierenden T-Lymphozyten, aus operativ oder bioptisch entnommenem Gewebe umfassen. Die (Immun-)Zellen können auch von Abstrichen und aus Aspiraten wie zum Beispiel Spülflüssigkeiten, Feinnadelaspiraten oder Sputum stammen. Die DNA kann auch aus Blut, Blutserum und Blutplasma stammen, beispielsweise in Form von frei zirkulierender DNA, exosomaler DNA, oder in Form von frei zirkulierenden Zellen der malignen Erkrankung und/oder peripheren Immunzellen, aus denen die DNA gewonnen wird. Die DNA kann auch aus weiteren Körperflüssigkeiten wie zum Beispiel Lymphflüssigkeit, Urin, Pleuraergüssen oder Aszites stammen, beispielsweise in Form von freier DNA oder in Form von Zellen der malignen Erkrankung oder Immunzellen, aus denen die DNA gewonnen wird. Die DNA kann auch aus nicht-konservierten (frischen) Zellen, Geweben und Körperflüssigkeiten, sowie aus fixierten Zellen, Geweben und Körperflüssigkeiten gewonnen werden. Die Fixierung der (Immun-)Zellen, Gewebe und Körperflüssigkeiten kann durch präzipitierende Fixative wie zum Beispiel Ethanol und andere Alkohole oder durch quervernetzende Fixative wie zum Beispiel Formaldehyd erreicht werden. Es kann sich zum Beispiel um Formalin-fixiertes und Paraffin-gebettetes Gewebe (FFPET) handeln. Die DNA kann auch aus beliebigen Kombinationen dieser Quellen stammen. Es kann sich auch um extrahierte DNA aus den vorgenannten Quellen handeln. Es ist auch möglich, die DNA anzureichern, zum Beispiel durch Fällung oder Extraktion. Dies kann sich zum Beispiel bei frei zirkulierender DNA aus den genannten Körperflüssigkeiten anbieten. Es ist auch möglich, die (Immun-)Zellen anzureichern, zum Beispiel durch Größenfiltration oder über Magnetpartikel, welche Antikörper auf der Oberfläche tragen, deren Antigene sich auf der Oberfläche der anzureichernden (Immun-)Zellen befinden. Dies kann sich zum Beispiel bei frei zirkulierenden Zellen der malignen Erkrankung oder Immunzellen aus den genannten Körperflüssigkeiten anbieten. Weitere geeignete Quellen für die zu analysierende DNA sind oder Homogenisate von Frischgeweben und Lysate von fixierten Geweben. Gegenüber herkömmlichen Prädiktionsverfahren, die auf immunohistochemischen Methoden oder einer mRNA-Expressionsanalyse beruhen, liegt ein besonderer Vorteil der vorliegenden Erfindung darin, dass die DNA-Methylierung selbst bei konservierten Probenmaterialien, geringsten Zellmengen oder gänzlich zellfreien DNA-Proben besonders robuste und akkurate Vorhersageergebnisse liefert.

In einer bevorzugten Variante umfasst die DNA deshalb frei zirkulierende DNA, DNA aus Exosomen und/oder DNA aus frei zirkulierenden (Immun-)Zellen aus einer Körperflüssigkeit, so genannte Flüssigbiopsien oder "liquid biopsies". Flüssigbiopsien stellen zurzeit ein zentrales Gebiet der onkologischen Forschung dar. Es wird dabei nicht das verdächtige Gewebe selbst, also zum Beispiel ein Tumorgewebe, sondern eine Probe einer Körperflüssigkeit analysiert, beispielsweise eine Blutprobe oder eine Lymphflüssigkeitsprobe. In dieser Probe können verschiedene Substanzen untersucht werden, die aus dem Tumor stammen, da frei zirkulierende genomische DNA, exosomale DNA, beziehungsweise frei zirkulierende Zellen oder Immunzellen vom Tumor in die Blutbahn freigesetzt werden. Es kann sich auch um Immunzellen handeln, die aus dem Thymus oder einem Lymphknoten kommen und in der Lage sind, spezifisch mit Zellen der malignen Erkrankung zu wechselwirken, aber nicht aus dem Tumor selbst stammen. Mit Vorteil wird das erfindungsgemäße Verfahren zur Analyse von Flüssigbiopsien verwendet, wenn der Tumor oder eine Metastase nicht biopsiert werden kann beziehungsweise wenn eine Biopsie für den Patienten im späten Tumorstadium eine zu große Gefahr darstellen würde. Es zeichnet die vorliegende Erfindung aus, dass die DNA-Methylierung der immunregulatorischen Gene sehr gut in Körperflüssigkeiten messbar ist, wohingegen ein herkömmlicher Expressionsnachweis der immunregulatorischen Gene anhand von mRNA oder Immunohistochemie nur schwer oder gar nicht möglich ist.

Die Umwandlung der DNA in Schritt B) kann im Prinzip mit allen im Stand der Technik für diesen Zweck bekannten und geeigneten Methoden erfolgen. Typischerweise handelt es sich um eine chemische oder enzymatische Umwandlung, beispielsweise durch Kontaktieren der DNA mit Bisulfit, zum Beispiel Natriumbisulfit oder Ammoniumbisulfit.

Erforderlichenfalls kann nach der Umwandlung in Schritt B) und vor der Untersuchung der DNA-Methylierung in Schritt C) ein Aufreinigen der DNA erfolgen. Geeignete Aufreinigungsmethoden und Protokolle sind dem Fachmann bekannt und können zum Beispiel eine DNA-Extraktion, eine Fällung oder eine polymergestützte Anreicherung (polymer-mediated enrichment) umfassen. Diesbezüglich wird auch auf die obigen Ausführungen verwiesen.

Mit der DNA-Methylierungsanalyse wird Anwesenheit, Abwesenheit oder Ausmaß einer DNA-Methylierung in dem analysierten Teil des immunregulatorischen Gens bestimmt. Der analysierte Teil enthält also mindestens ein CpG-Dinukleotid, welches auf DNA-Methylierung untersucht wird, vorzugsweise mehrere CpG-Dinukleotide, welche auf DNA-Methylierung untersucht werden. Anwesenheit der DNA-Methylierung bedeutet demnach, dass in dem analysierten Teil des immunregulatorischen Gens mindestens ein methyliertes CpG-Dinukleotid nachgewiesen wird. Abwesenheit der DNA-Methylierung bedeutet, dass bei keinem der in dem Teil enthaltenen CpG-Dinukleotide eine Methylierung nachweisbar ist. Die Bestimmung des Ausmaßes der DNA-Methylierung des immunregulatorischen Gens kann eine Untersuchung mehrerer CpG-Dinukleotide auf DNA-Methylierung umfassen, die in dem analysierten Teil enthalten sind. Die Bestimmung des Ausmaßes der DNA-Methylierung des immunregulatorischen Gens kann auch eine Untersuchung des gleichen CpG-Dinukleotids auf DNA-Methylierung in einer Mehrzahl von Genkopien des immunregulatorischen Gens umfassen. Auch Kombinationen dieser Varianten sind möglich.

Die Untersuchung der DNA-Methylierung des immunregulatorischen Gens in Schritt C) unterliegt dabei keinen besonderen Beschränkungen. Geeignete Methoden kann ein Fachmann unschwer auf der Grundlage dieser Offenbarung bestimmen. Diesbezüglich wird auch auf die oben genannten Laborhandbücher verwiesen. In einer bevorzugten Variante wird zunächst eine Polymerasekettenreaktion (PCR) mit Oligonukleotiden, sogenannten Primern, durchgeführt, welche dazu ausgelegt ist, einen Abschnitt der in Schritt B) umgewandelten DNA zu amplifizieren, welcher den zu analysierenden Teil des immunregulatorischen Gens beziehungsweise das mindestens eine zu analysierende CpG-Dinukleotid umfasst. Anschließend erfolgt vorzugsweise eine Sequenzierung zumindest eines Teils des Amplifikats, zum Beispiel eine Sanger-Sequenzierung, Pyrosequenzierung, massenspektrometrische Sequenzierung oder eine Sequenzierung der zweiten oder dritten Generation, welche auch als "Massive Parallel Sequencing", "Next Generation Sequencing" (NGS) oder als Nanoporensequenzierung bezeichnet werden. Es ist auch möglich, im Anschluss an die PCR eine Hybridisierung mit methylierungsspezifischen Oligonukleotiden (Sonden) durchzuführen, beispielsweise in Form eines DNA-Microarrays. Die DNA-Methylierung kann auch durch quantitative Echtzeit-PCR (quantitative real-time PCR, qPCR), gegebenenfalls gefolgt von einer Schmelzkurvenanalyse, bestimmt werden. Insbesondere kann die quantitative Echtzeit-PCR mit methylierungsspezifischen Primern wie in WO 1997/046705 A1 und/oder methylierungsspezifischen Blockeroligonukleotiden wie in WO 2002/072880 A2 durchgeführt werden. In einer bevorzugten Variante werden methylierungsspezifische Detektionssonden verwendet.

In wiederum anderen bevorzugten Varianten kann eine PCR entfallen, beispielsweise bei einem "Whole Genome Shotgun Bisulfite Sequencing" (WGSBS) oder einer direkten Nanoporensequenzierung. Bei der WGSBS wird die DNA fragmentiert, anschließend werden Adapter an die DNA Fragmente ligiert. Über die Adapter ist im Anschluss eine Amplifikation und Sequenzierung möglich. Es ist auch möglich, bei der WGSBS auf den Schritt der Fragmentierung zu verzichten, da die DNA zum Beispiel durch die Umwandlung durch Bisulfit-Behandlung bereits fragmentiert vorliegen kann. Protokolle für die Durchführung einer WGSBS sind dem Fachmann leicht zugänglich (Johnson, M. D. et al., Curr. Protoc. Mol. Biol., 2012, 99, 21.23.1-21.23.28; Lister, R. et al., Nature, 2009, 462, 315-322; Berman, B. P. et al., Nat. Genet., 2011, 44, 40-46).

In einer anderen bevorzugten Variante kann vor der PCR Amplifikation eine Hybridisierung mit spezifischen Oligonukleotiden (Sonden) erfolgen, die im Falle des Bindens ligiert werden und anschließend mittels PCR amplifiziert werden. Geeignete Methoden und Protokolle, wie zum Beispiel eine "multiplex ligation dependent probe amplification" (MLPA) stehen dem Fachmann ohne Probleme zur Verfügung, zum Beispiel in "PCR Mutation Detection Protocols" von B. D. M. Theophilus und R. Rapley, 2nd Edition, 2011, Springer.

In einer weiteren bevorzugten Variante wird die DNA-Methylierungsanalyse mittels des Infinium HumanMethylation450 BeadChip durchgeführt. Geeignete Protokolle finden sich beispielsweise im Kapitel "Determination of DNA Methylation Levels Using Illumina HumanMethylation450 BeadChips" von M.A. Carless, welches im Buch "Chromatin Protocols" von S.P. Chellappan, Band 1288, 2015, der Buchserie "Methods in Molecular Biology", Springer Science+Business Media New York, zu finden ist. Weitere geeignete Protokolle sind aus den nachfolgenden Ausführungsbeispielen ersichtlich.

Der Erfinder hat herausgefunden, dass das Ansprechen der malignen Erkrankung auf die Immuntherapie umso wahrscheinlicher ist, je geringer das Ausmaß der DNA-Methylierung des immunregulatorischen Gens ist. Folglich ist das Ansprechen der malignen Erkrankung auf die Immuntherapie besonders wahrscheinlich, wenn die DNA-Methylierung des immunregulatorischen Gens gegen Null geht, also insbesondere bei tatsächlicher oder aufgrund von technischen Nachweisgrenzen zumindest faktischer Abwesenheit der DNA-Methylierung. Es ist daher von Vorteil, wenn die DNA-Methylierungsanalyse unter Bedingungen durchgeführt wird, welche eine quantitative Bestimmung der DNA-Methylierung des immunregulatorischen Gens erlauben. Wenn zum Beispiel die DNA-Methylierungsanalyse Genkopien des immunregulatorischen Gens von mehreren Zellen der malignen Erkrankung und/oder von mehreren der mit den Zellen der malignen Erkrankung wechselwirkenden Immunzellen umfasst, kann ein Anteil derjenigen Genkopien des immunregulatorischen Gens bestimmt werden, welche die DNA-Methylierung aufweisen. Zu diesem Zweck kann die Anzahl oder Menge der methylierten Genkopien des immunregulatorischen Gens zu der Gesamtzahl oder Gesamtmenge der analysierten Genkopien des immunregulatorischen Gens in ein Verhältnis gesetzt. Auf diese Weise kann ein Ansprechen der malignen Erkrankung auf die Immuntherapie noch genauer vorhergesagt werden und beispielsweise wahrscheinlich sein, wenn kleiner gleich 40%, kleiner gleich 35%, kleiner gleich 30%, kleiner gleich 25%, kleiner gleich 20%, kleiner gleich 15%, kleiner gleich 10% oder kleiner gleich 5% der Genkopien des immunregulatorischen Gens die DNA-Methylierung aufweisen. In wiederum anderen Varianten ist es möglich, dass die maligne Erkrankung auf die Immuntherapie wahrscheinlich nicht anspricht, wenn mehr als 30%, mehr als 35%, mehr als 40% oder mehr als 45% der Genkopien des immunregulatorischen Gens die DNA-Methylierung aufweisen oder prinzipiell bei Anwesenheit der DNA-Methylierung.

Bei den mit den Zellen der malignen Erkrankung wechselwirkenden Immunzellen kann es sich um T-Lymphozyten, B-Lymphozyten, antigenpräsentierende Zellen oder natürlichen Killerzellen (NKs) handeln. Auch Kombinationen dieser Immunzellen sind möglich. In bestimmten Varianten umfassen die Immunzellen tumorinfiltrierende T-Lymphozyten und/oder B-Lymphozyten, insbesondere tumorinfiltrierende CD8⁺-Lymphozyten und/oder regulatorische T-Lymphozyten (Tregs). Es kann sich aber auch um periphere und/oder lympatische T-Lymphozyten, B-Lymphozyten, antigenpräsentierende Zellen und/oder NKs handeln.

Die DNA-Methylierungsanalyse kann einen Teil oder mehrere Teile beziehungsweise ein oder mehrere CpG-Dinukleotide innerhalb und in der Umgebung des immunregulatorischen Gens umfassen. Vorzugsweise umfasst die DNA-Methylierungsanalyse zumindest einen Teil eines regulatorischen Genbereichs, insbesondere einer Transkriptionsfaktorbindestelle, eines Promotors, einer CpG-Insel, eines Silencers, eines Enhancers, oder einer CTCF-Bindestelle. Die DNA-Methylierungsanalyse kann auch zumindest einen Teil einer für ein Transkript des immunregulatorischen Gens codierenden Sequenz umfassen. Auch Kombinationen der vorgenannten Teile sind möglich. Enhancer können beispielsweise vom Gen entfernt als distale Enhancer vorliegen. Enhancer können auch in der Nähe des Gens liegen und werden dann proximale Enhancer benannt. Regulatorische Genbereiche sind dem Fachmann gut bekannt und beispielsweise in "Gene Control" von D. S. Latchman, 2. Edition, 2015, Garyland Science, Taylor & Francis Group, LLC, beschrieben. Für die erfindungsgemäße Verfahrensausführung sind beispielsweise auch diejenigen Teile beziehungsweise CpG-Dinukleotide geeignet, deren Methylierungszustand mit der transkriptionellen Aktivität beziehungsweise der Expression des immunregulatorischen Gens korreliert. Die transkriptionelle Aktivität ist beispielsweise anhand einer veränderten Chromatinstruktur ersichtlich. Sogenanntes "offenes Chromatin" kann mit einer hohen transkriptionellen Aktivität eines Gens einhergehen wie beispielsweise in "Genetik" von W. Janning und E. Kunst, 2004, Georg Thieme Verlag, Stuttgart und New York, beschrieben ist. Bereiche "offenen Chromatins" sind daher für die erfindungsgemäße DNA-Methylierungsanalyse geeignet.

Die Bestimmung regulatorischer Genelemente ist für den Fachmann ohne Weiteres anhand geeigneter Datenbanken möglich. Beispielsweise sind in der Datenbank "Ensembl" solche regulatorischen Elemente annotiert, wie beispielsweise in "The Ensembl Regulatory Build" von D. R. Zerbino, S. P. Wilder, N. Johnson, T. Juettemann und P. R. Flicek, 2015, Genome Biology, Ausgabe 16, doi:10.1186/s13059-015-0621-5 beschrieben.

Eine geeignete Primärsequenz des humanen Genoms, anhand derer geeignete und bevorzugte Bereiche und Sequenzen der immunregulatorischen Gene für die erfindungsgemäße DNA-Methylierungsanalyse bestimmt werden können, ist beispielsweise die humane Genomversion des Genome Reference Consortium Genome Reference Consortium Human Build 38 (GRCh38) beziehungsweise Reference Consortium Human Build 38 patch release 10 (GRCh38.p10) mit Stand vom 15. Oktober 2017. Auf Bereiche des Genoms wird nachfolgend in der Schreibweise "Chromosomennummer:Position der ersten Base des Bereichs-Position der letzten Base des Bereichs" Bezug genommen, also beispielsweise "2:203583059-203583108" für den Bereich von Base 203583059 bis Base 203583108 von Chromosom 2.

Die DNA-Methylierungsanalyse des *CTLA4* Gens umfasst bevorzugt zumindest einen Teil eines oder mehrerer der folgenden Bereiche: einen Bereich, der das Gen *CTLA4* und die benachbarten komethylierten Gene *CD28* und *ICOS* umfasst (2:203551590-204126647, SEQ ID NO:1), einen Bereich, der für die Transkripte codiert (2:203867786-203873960), einen Promotor (2:203866174-203868926 und 2:203869477-203874095), einen Enhancer (2:203874152-203875266, 2:203876672-203878051 und 2:203879313-203881585), einen Bereich zwischen *CTLA4* und dem benachbarten immunregulatorischen Gen *ICOS* (2:203872383-203939876), einen Bereich zwischen *CTLA4* und dem benachbarten immunregulatorischen Gen *CD28* (2:203738066-203867984), einen oder mehrere Bereiche ausgewählt aus SEQ ID NO:51, SEQ ID NO:27, SEQ ID NO:48, SEQ ID NO:28, SEQ ID NO:29, SEQ ID NO:50, SEQ ID NO:32, SEQ ID NO:33, SEQ ID NO:30, SEQ ID NO:49, SEQ ID NO:31 und SEQ ID NO:52.

Die DNA-Methylierungsanalyse des *CD28* Gens umfasst bevorzugt zumindest einen Teil eines oder mehrerer der folgenden Bereiche: einen Bereich, welcher eine codierenden Sequenz von *CD28* und einen Promotor umfasst (2:203551590-203754454), einen Bereich, welcher die codierende Sequenz beinhaltet (2:203706475-203738912) und einen Bereich, welcher einen Promotor umfasst (2:203677265-203707326).

Die DNA-Methylierungsanalyse des *ICOS* Gens umfasst bevorzugt zumindest einen Teil eines oder mehrerer der folgenden Bereiche: einen Bereich, welcher für ein Transkript codiert (2:203936748-203961577), einen Promotor (2:203934590-203941036 und 2:203948548-203953636), einen Enhancer (2:203931099-203937863 und 2:203940518-203949061), einen oder mehrere Bereiche ausgewählt aus SEQ ID NO:52, SEQ ID NO:53, SEQ ID NO:34, SEQ ID NO:54, SEQ ID NO:36, SEQ ID NO:56, SEQ ID NO:35 und SEQ ID NO:55.

Die DNA-Methylierungsanalyse des *CD86* Gens umfasst bevorzugt zumindest einen Teil eines oder mehrerer der folgenden Bereiche: einen Bereich, welcher eine codierenden Sequenz von *CD86* und einen Promotor umfasst (3:122039741-122154807, SEQ ID NO:3), einen Bereich, welcher für Transkripte codiert (3:122054701-122121475), einen Promotor (3:122054261-122061082, 3:122073513-122079893 und 3:122087703-122093314), einen Enhancer (3:122098924-122104974), einen oder mehrere Bereiche ausgewählt aus SEQ ID NO:65, SEQ ID NO:67, SEQ ID NO:66, SEQ ID NO:70, SEQ ID NO:72, SEQ ID NO:69, SEQ ID NO:75, SEQ ID NO:73, SEQ ID NO:74, SEQ ID NO:71, SEQ ID NO:68, SEQ ID NO:76, SEQ ID NO:77.

Die DNA-Methylierungsanalyse des *CD80* Gens umfasst bevorzugt zumindest einen Teil eines oder mehrerer der folgenden Bereiche: einen Bereich, welcher eine codierenden Sequenz von *CD80* und eine Promotor umfasst (3:119523584-119573836, SEQ ID NO:2), einen Bereich, welcher für Transkripte codiert (3:119524293-119559602), einen Promotor (3:119554042-119563668 und 3:119568227-119573274), einen Enhancer (3:119563379-119568778, 3:119538188-119543511 und 3:119545840-119554325 ), einen oder mehrere Bereiche ausgewählt aus SEQ ID NO:57, SEQ ID NO:61, SEQ ID NO:62, SEQ ID NO:60, SEQ ID NO:59, SEQ ID NO:64, SEQ ID NO:58 und SEQ ID NO:63.

In einer bevorzugten Variante umfasst die DNA-Methylierungsanalyse zumindest einen Teil von *CTLA4.* In einer weiteren bevorzugten Variante umfasst die DNA-Methylierungsanalyse zumindest einen Teil von *CD86.* In noch einer weiteren bevorzugten Variante umfasst die DNA-Methylierungsanalyse zumindest einen Teil von *CD28.* In noch einer weiteren bevorzugten Variante umfasst die DNA-Methylierungsanalyse zumindest einen Teil von *CD80.* In noch einer weiteren bevorzugten Variante umfasst die DNA-Methylierungsanalyse zumindest einen Teil von *ICOS.* In besonders bevorzugten Varianten umfasst die DNA-Methylierungsanalyse zumindest jeweils einen Teil von mindestens zwei der immunregulatorischen Gene. Auf diese Weise wird eine zuverlässige Vorhersage des Ansprechverhaltens der malignen Erkrankung erreicht, insbesondere wenn nur geringe Mengen an DNA für die DNA-Methylierungsanalyse zur Verfügung stehen. Diesbezüglich wird auch auf das nachfolgende Ausführungsbeispiel 2 verwiesen. Bevorzugt umfasst die DNA-Methylierungsanalyse zumindest einen Teil von *CTLA4* und zumindest einen Teil von mindestens einem weiteren der immunregulatorischen Gene.

Die DNA-Methylierungsanalyse kann auch zusätzlich zu den vorgenannten Genen weitere immunregulatorische Gene umfassen, um durch eine Kombination der Ergebnisse eine noch genauere Subtypisierung von Patienten zu erreichen. Insbesondere kann die DNA-Methylierungsanalyse zusätzlich zumindest einen Teil von *PDCD1, CD274* und/oder *PDCD1LG2* umfassen, welche für die Immun-Checkpoints PD-1, PD-L1 und PD-L2 codieren. Beispielsweise kann die DNA-Methylierungsanalyse zumindest einen Teil von *CTLA4* und zumindest einen Teil von *PDCD1, CD274* und/oder *PDCD1LG2* umfassen. Die DNA-Methylierungsanalyse kann auch zumindest einen Teil von *CTLA4* und zumindest einen Teil von *PDCD1, CD274, PDCD1LG2, CD86, CD28, CD80* und/oder *ICOS* umfassen.

Die DNA-Methylierungsanalyse des *PDCD1* Gens umfasst bevorzugt zumindest einen Teil eines oder mehrerer der folgenden Bereiche: einen Bereich, welcher für ein Transkript codiert (2:241849881-241858908), einen Bereich mit offenem Chromatin (2:241849051-241853001 und 2:241861820-241862593), einen Enhancer (2:241852997-241855201), eine CTCF-Bindestelle (2:241859081-241860074), einen Promotor (2:241856912-241861429 und 2:241862929-241865230).

Die DNA-Methylierungsanalyse des *CD274* Gens umfasst bevorzugt zumindest einen Teil eines oder mehrerer der folgenden Bereiche: einen Bereich, welcher für ein Transkript codiert (9:5450503-5470566), einen Promotor (9:5445402-5456799 und 9:5458041-5461360), einen Enhancer (9:5457122-5457702, 9:5463574-5468340, 9:5440647-5441785 und 9:5472191-5473149), eine CTCF-Bindestelle (9:5440970-5441435 und 9:5446325-5446870).

Die DNA-Methylierungsanalyse des *PDCD1LG2* Gens umfasst bevorzugt zumindest einen Teil eines oder mehrerer der folgenden Bereiche: einen Bereich, welcher für ein Transkript codiert (9:5510570-5571254), einen Promotor (9:5507688-5523442, 9:5491444-5503289, 9:5528150-5534251 und 9:5547972-5571492), einen Enhancer (9:5479110-5491616, 9:5522642-5528253, 9:5534822-5547690 und 9:5572730-5580962), einen Bereich vor der codierenden Sequenz (9:5496357-5510570).

Die vorgenannten Merkmale und bevorzugten Ausgestaltungen des ersten Aspektes sind ebenfalls vollumfänglich in den nachfolgenden Erfindungsaspekten enthalten und werden lediglich nicht erneut erwähnt, um Wiederholungen zu vermeiden.

In der personalisierten onkologischen Medizin sind die Therapien auf Patienten mit spezifischen Krebstypen abgestimmt. Eine hohe Ansprechrate von Patienten auf eine zielgerichtete Immuntherapie mit einem PD-1-Immun-Checkpoint Inhibitor ist nur zu erreichen, wenn Patienten mit entsprechend sensitiven Erkrankungen zuverlässig erkannt und gezielt für die Behandlung ausgewählt werden. In diesem Sinne wird gemäß dem zweiten Erfindungsaspekt ein Verfahren angegeben, mit dem ein an einer malignen Erkrankung leidender Patient für eine Immuntherapie ausgewählt wird, die dazu ausgelegt ist, einen PD-1-Immun-Checkpoint-Signalweg zu inhibieren. Das Verfahren umfasst die folgenden Schritte: A) Bereitstellen von Zellen der malignen Erkrankung und/oder von mit den Zellen der malignen Erkrankung wechselwirkenden Immunzellen des Patienten, B) Durchführen einer DNA-Methylierungsanalyse zumindest eines Teils eines immunregulatorischen Gens ausgewählt aus *CTLA4, CD86, CD28, CD80* und/oder *ICOS* von den Zellen der malignen Erkrankung und/oder von mit den Zellen der malignen Erkrankung wechselwirkenden Immunzellen aus A), C) Auswählen des Patienten für die Immuntherapie anhand von Anwesenheit, Abwesenheit und/oder Ausmaß einer in B) ermittelten DNA-Methylierung des immunregulatorischen Gens, wobei die Immuntherapie einen Wirkstoff umfasst, welcher den PD-1-Immun-Checkpoint-Signalweg durch eine Bindung des Wirkstoffs an PD-1, PD-L1 und/oder PD-L2 inhibiert, wobei der Wirkstoff einen anti-PD-1 Antikörper, einen anti-PD-L1 Antikörper und/oder einen anti-PD-L2 Antikörper umfasst.

Insbesondere wird der Patient ausgewählt, wenn die DNA-Methylierung des immunregulatorischen Gens anzeigt, dass das Ansprechen der malignen Erkrankung auf die Immuntherapie wahrscheinlich ist. Diesbezüglich wird auf die entsprechenden Ausführungen zum ersten Aspekt verwiesen.

Gemäß einem dritten Erfindungsaspekt kann eine DNA-Methylierungsanalyse zumindest eines Teils eines immunregulatorischen Gens ausgewählt aus *CTLA4, CD86, CD28, CD80* und/oder *ICOS* von Zellen einer malignen Erkrankung und/oder von mit den Zellen der malignen Erkrankung wechselwirkenden Immunzellen in verschiedener Weise in der personalisierten Medizin verwendet werden. Möglich ist zum Beispiel eine Verwendung zur Vorhersage eines Ansprechens der malignen Erkrankung auf eine Immuntherapie, eine Verwendung zur individualisierten Auswahl einer Immuntherapie für die malignen Erkrankung, und/oder eine Verwendung zur Auswahl eines Patienten, welcher an der malignen Erkrankung leidet, für eine Immuntherapie, wenn die Immuntherapie dazu ausgelegt ist, einen PD-1-Immun-Checkpoint-Signalweg zu inhibieren und einen Wirkstoff umfasst, welcher den PD-1-Immun-Checkpoint-Signalweg durch eine Bindung des Wirkstoffs an PD-1, PD-L1 und/oder PD-L2 inhibiert, wobei der Wirkstoff einen anti-PD-1 Antikörper, einen anti-PD-L1 Antikörper und/oder einen anti-PD-L2 Antikörper umfasst.

Ein vierter Aspekt der Erfindung betrifft die Verwendung von Anwesenheit, Abwesenheit oder Ausmaß einer DNA-Methylierung von zumindest einem Teil eines immunregulatorischen Gens ausgewählt aus *CTLA4, CD86, CD28, CD80* und/oder *ICOS* von Zellen einer malignen Erkrankung und/oder von mit den Zellen der malignen Erkrankung wechselwirkenden Immunzellen als Biomarker zur Vorhersage eines Ansprechens der malignen Erkrankung auf eine Immuntherapie, zur individualisierten Auswahl einer Immuntherapie für die malignen Erkrankung, und/oder zur Auswahl eines Patienten, welcher an der malignen Erkrankung leidet, für eine Immuntherapie, wobei die Immuntherapie jeweils dazu ausgelegt ist, einen PD-1-Immun-Checkpoint-Signalweg zu inhibieren und einen Wirkstoff umfasst, welcher den PD-1-Immun-Checkpoint-Signalweg durch eine Bindung des Wirkstoffs an PD-1, PD-L1 und/oder PD-L2 inhibiert, wobei der Wirkstoff einen anti-PD-1 Antikörper, einen anti-PD-L1 Antikörper und/oder einen anti-PD-L2 Antikörper umfasst.

In den vorgenannten Verwendungen kann die Immuntherapie auch zusätzlich dazu ausgelegt sein, einen CTLA4-Immun-Checkpoint-Signalweg zu inhibieren. Der Erfinder hat in seinen klinischen Studien nicht nur erkannt, dass die DNA-Methylierungsanalyse der erfindungsgemäßen Gene ebenso zuverlässig ein wahrscheinliches Ansprechen der malignen Erkrankung auf solche CTLA4-Immun-Checkpoint-Signalweg inhibierenden Immuntherapien anzeigt, sondern auch, dass die Verbindung von PD-1- und CTLA4-Immun-Checkpoint Inhibition im Rahmen einer Immuntherapie mit besonderem Behandlungserfolg verbunden sein kann. Es zeichnet die Erfindung deshalb aus, dass sie dem Kliniker mithilfe eines einzigen diagnostischen Test durch Anwesenheit, Abwesenheit oder Ausmaß der DNA-Methylierung des immunregulatorischen Gens mindestens drei Therapieoptionen aufzeigen kann: eine selektive Inhibierung des PD-1-Immun-Checkpoint-Signalwegs (zum Beispiel als Monotherapie), eine kombinierte Inhibierung der PD-1- und CTLA4-Immun-Checkpoint-Signalwege sowie eine sequenzielle Inhibierung zunächst des PD-1-Immun-Checkpoint-Signalwegs und anschließend des CTLA4-Immun-Checkpoint-Signalwegs. Letztere Option kann sich zum Beispiel anbieten, wenn die Inhibierung des PD-1-Immun-Checkpoint-Signalwegs starke Nebenwirkungen bei einem Patienten verursacht und deshalb abgesetzt werden muss oder wenn die PD-1-Inhibition nicht mehr wirkt und der Patient einen Progress der Erkrankung zeigt.

Die auf die Inhibierung des CTLA4-Immun-Checkpoint-Signalwegs ausgelegte Immuntherapie umfasst vorzugsweise einen Wirkstoff, welcher den CTLA4-Immun-Checkpoint-Signalweg durch eine Bindung des Wirkstoffs an CTLA4, CD80, CD86 oder CD28 inhibiert. Die Immuntherapie beziehungsweise der Wirkstoff kann zum Beispiel einen anti-CTLA4 Antikörper, einen anti-CD80 Antikörper, einen anti-CD86 Antikörper und/oder einen anti-CD28 Antikörper umfassen. Insbesondere handelt es sich dabei um monoklonale Antikörper.

Ein fünfter Aspekt der Erfindung betrifft die Verwendung eines Kits zur Durchführung der Verfahren nach dem ersten oder zweiten Aspekt. Das Kit umfasst Reagenzien für die DNA-Methylierungsanalyse zumindest eines Teils eines immunregulatorischen Gens ausgewählt aus *CTLA4, CD86, CD28, CD80* und/oder *ICOS* von Zellen einer malignen Erkrankung und/oder von mit den Zellen der malignen Erkrankung wechselwirkenden Immunzellen, um Anwesenheit, Abwesenheit und/oder Ausmaß der DNA-Methylierung des immunregulatorischen Gens zu bestimmen. Das Kit kann auch Instruktionen zur Vorhersage des Ansprechens der malignen Erkrankung auf die Immuntherapie anhand von Anwesenheit, Abwesenheit und/oder Ausmaß der DNA-Methylierung des immunregulatorischen Gens umfassen.

Insbesondere enthält das Kit mindestens ein erstes Oligonukleotid-Paar für die DNA-Methylierungsanalyse, welches dazu ausgelegt ist, an eine Sequenz des immunregulatorischen Gens in DNA von den Zellen der malignen Erkrankung und/oder von den Immunzellen zu hybridisieren, nachdem in der DNA enthaltene Cytosine in Uracil oder eine andere Base mit einem von Cytosin unterscheidbaren Basenpaarungsverhalten und/oder Molekulargewicht umgewandelt wurden, um die Sequenz zu amplifizieren und/oder zu detektieren. Mindestens eines der Oligonukleotide kann so ausgelegt sein, dass es zwischen umgewandelter methylierter und umgewandelter unmethylierter DNA unterscheidet, sodass die Sequenz methylierungsabhängig amplifiziert wird. Dafür kann das Oligonukleotid revers-komplementär zu einer Bindungssequenz sein, welche mindestens ein zu analysierendes CpG-Dinukleotid enthält. Das Oligonukleotid kann zum Beispiel revers-komplementär zu der Bindungssequenz sein, wenn das Cytosin in dem CpG-Dinukleotid umgewandelt wurde, also ursprünglich unmethyliert vorlag. Alternativ kann das Oligonukleotid revers-komplementär zu der Bindungssequenz sein, wenn das Cytosin in dem CpG nicht umgewandelt wurde, also ursprünglich methyliert vorlag. Auf diese Weise wird erreicht, dass eine Amplifikation nur stattfindet, wenn die Sequenz methyliert beziehungsweise unmethyliert vorliegt.

Es ist auch möglich, dass die Oligonukleotide dazu ausgelegt sind, die Sequenz unabhängig von einer DNA-Methylierung zu amplifizieren. Vorzugsweise sind die Oligonukleotide dann revers-komplementär zu Bindungssequenzen, welche keine zu analysierenden CpG-Dinukleotide enthalten. Vorzugsweise befinden sich die zu analysierenden CpG-Dinukleotide zwischen den Bindungssequenzen der Oligonukleotide. Das Kit kann zusätzlich Hybridisierungssonden enthalten, welche zwischen umgewandelter methylierter Sequenz und umgewandelter unmethylierter Sequenz unterscheiden, sodass die amplifizierte Sequenz methylierungsabhängig detektiert wird. Aus dem Signalverhältnis der Sonden lässt sich dann das Ausmaß der DNA-Methylierung ablesen.

Weiterhin kann das Kit mindestens ein zweites Oligonukleotid-Paar umfassen, das dazu ausgelegt ist, an eine Sequenz der umgewandelten DNA zu hybridisieren, welche keine CpG-Dinukleotide enthält, um die Sequenz methylierungsunabhängig zu amplifizieren und/oder zu detektieren. Mithilfe eines derart ausgelegten Oligonukleotid-Paars lässt sich zum Beispiel die Gesamtzahl beziehungsweise die Gesamtmenge der Genom- oder Genkopien, insbesondere der Genkopien des immunregulatorischen Gens, bestimmen, die in der umgewandelten DNA vorliegen. Auf diese Weise kann gemäß obiger Ausführungen zum ersten Aspekt der relative Anteil derjenigen Genkopien des immunregulatorischen Gens bestimmt werden, welche die DNA-Methylierung aufweisen. Diesbezüglich wird auch auf das erste Ausführungsbeispiel verwiesen.

In bevorzugten Varianten enthält das Kit zwei oder mehrere erste Oligonukleotidpaare, welche dazu ausgelegt sind, an Sequenzen von mindestens zwei verschiedenen immunregulatorischen Genen ausgewählt aus *CTLA4, CD86, CD28, CD80* und/oder *ICOS* in der umgewandelten DNA zu hybridisieren, um die Sequenzen für die DNA-Methylierungsanalyse zu amplifizieren und/oder zu detektieren. Durch die kombinierte DNA-Methylierungsanalyse von mindestens zwei der erfindungsgemäßen immunregulatorischen Gene lässt sich ein besonders robuster Messwert und somit eine besonders zuverlässige Vorhersage des Ansprechens auf die Immuntherapie erreichen. Diesbezüglich wird auch auf das zweite Ausführungsbeispiel verwiesen.

Bevorzugte Bereiche und Sequenzen des immunregulatorischen Gens, welche mithilfe der Oligonukleotid-Paare zu amplifizieren und/oder zu detektieren sind, entsprechen denen des ersten Aspektes.

Das Kit umfasst vorzugsweise eine Gebrauchsanweisung für die Durchführung des Verfahrens nach dem ersten und/oder zweiten Aspekt und/oder für die Verwendung nach dem dritten oder vierten Aspekt.

Alle vorgenannten Erfindungsaspekte betreffen deduktive Schritte in Zusammenhang mit einem vorausgehenden *in vitro* Verfahren, sodass kein erfindungswesentlicher technischer Schritt am menschlichen oder tierischen Körper stattfindet.

Es ist aber prinzipiell auch möglich, das erfindungsgemäße Verfahren in einen maßgeschneiderten Behandlungsansatz für einen Patienten mit einer malignen Erkrankung einfließen zu lassen, um die Wahrscheinlichkeit eines Therapie-Ansprechens zu verbessern. Folglich betrifft der sechste Aspekt der Erfindung ein Verfahren zur immuntherapeutischen Behandlung eines Patienten, welcher an einer malignen Erkrankung leidet, mit einem Wirkstoff, welcher dazu ausgelegt ist, einen PD-1-Immun-Checkpoint-Signalweg zu inhibieren. Bei dem Verfahren wird in Schritt I) vor und/oder während der immuntherapeutischen Behandlung eine DNA-Methylierungsanalyse zumindest eines Teils eines immunregulatorischen Gens ausgewählt aus *CTLA4, CD86, CD28, CD80* und/oder *ICOS* von Zellen der malignen Erkrankung und/oder von mit den Zellen der malignen Erkrankung wechselwirkenden Immunzellen durchgeführt und anhand von Anwesenheit, Abwesenheit und/oder Ausmaß einer DNA-Methylierung des immunregulatorischen Gens das Ansprechen der malignen Erkrankung auf den Wirkstoff vorhergesagt. Anschließend wird in Schritt II) der Wirkstoff verabreicht, wenn Schritt I) anzeigt, dass die maligne Erkrankung bei der immuntherapeutischen Behandlung mit dem Wirkstoff wahrscheinlich anspricht. Wenn I) allerdings anzeigt, dass die maligne Erkrankung bei der immuntherapeutischen Behandlung mit dem Wirkstoff wahrscheinlich nicht oder nicht mehr anspricht, wird alternativ zu Schritt II) der Wirkstoff nicht verabreicht oder eine Verabreichung des Wirkstoffs wird beendet oder reduziert (Schritt III). Bezüglich des wahrscheinlichen Ansprechens wird auf die Ausführungen zum ersten Aspekt verwiesen. Auf diese Weise trägt die Erfindung durch diagnostische Tests zur besseren Typisierung der malignen Erkrankung bei und ermöglicht so eine maßgeschneiderte Behandlung mit verbessertem Nutzen-Risiko-Verhältnis.

Bei der Behandlung kann in Schritt II) zusätzlich zum Wirkstoff und/oder in Schritt III) anstatt des Wirkstoffs ein weiterer beziehungsweise anderer Wirkstoff verabreicht werden, welcher dazu ausgelegt ist, einen CTLA4-Immun-Checkpoint-Signalweg zu inhibieren. Wie bereits zum vierten Aspekt ausgeführt wurde, liegt ein besonderer Vorteil der Erfindung darin, dass dem Kliniker mithilfe eines einzigen diagnostischen Tests sowohl komplementäre als auch alternative immuntherapeutische Behandlungsoptionen aufgezeigt werden können: eine selektive Inhibierung des PD-1-Immun-Checkpoint-Signalwegs oder alternativ des CTLA4-Immun-Checkpoint-Signalwegs, eine kombinierte Inhibierung der PD-1- und CTLA4-Immun-Checkpoint-Signalwege sowie eine sequenzielle Inhibierung zunächst des PD-1-Immun-Checkpoint-Signalwegs und anschließend des CTLA4-Immun-Checkpoint-Signalwegs. Auf diese Weise kann der Kliniker die Behandlungsmöglichkeiten für die maligne Erkrankung noch gezielter und effizienter ausschöpfen.

Der weitere beziehungsweise andere Wirkstoff kann zum Beispiel einen anti-CTLA4 Antikörper, einen anti-CD80 Antikörper, einen anti-CD86 Antikörper und/oder einen anti-CD28 Antikörper umfassen. Insbesondere kann es sich dabei um einen monoklonalen Antikörper handeln.

### Detaillierte Beschreibung von Ausführungsbeispielen

Nachfolgend wird die Erfindung anhand von Ausführungsbeispielen und experimentellen Ergebnissen näher beschrieben. Diese Ausführungsbeispiele dienen der Erläuterung und nicht der Beschränkung auf spezifische Details.

### Beispiel 1: Klinische Studie zur Vorhersage des Ansprechens einer malignen Erkrankung auf eine Inhibierung des PD-1-Immun-Checkpoint-Signalwegs anhand der DNA-Methylierung von CTLA4

Die untersuchte Patientenkohorte umfasste insgesamt 50 Patienten, bei denen metastasierte maligne Melanome diagnostiziert wurden. Vor Beginn der immuntherapeutischen Behandlung wurden Tumorgewebeproben der Patienten genommen, durch Formalin fixiert und in Paraffin eingebettet. Die Patienten wurden zwischen Oktober 2014 und April 2017 mit einer anti-PD-1 Immun-Checkpoint Blockade durch Pembrolizumab oder Nivolumab behandelt.

Für die DNA-Methylierungsanalyse wurde zunächst von den Tumorgewebeproben Dünnschnitte mit einer Stärke von 10 µm angefertigt und auf Glasobjektträger aufgezogen. Anhand eines HE-Schnitts wurden die Tumorareale durch pathologische Begutachtung identifiziert und mit einem Skalpell von den Glasobjekträgern zur weiteren Behandlung heruntergekratzt. Bisulfit-konvertierte DNA wurde aus den Tumorarealen mit dem innuCONVERT Bisulfit All-In-One Kit (Analytik Jena, Jena, Deutschland) nach Herstellerangaben bereitgestellt. Anschließend wurde die Gesamtmenge konvertierter DNA mithilfe eines NanoDrop ND-1000 Spektrophotometers (Thermo Fisher Scientific, Waltham, MA, USA) quantifiziert.

Im nächsten Schritt wurde eine DNA-Methylierungsanalyse durchgeführt, indem beispielsweise ein Teil des *CTLA4* Genlokus methylierungsspezifisch mithilfe einer quantitativen Echtzeit-PCR amplifiziert und gleichzeitig quantifiziert wurde. Dabei kam zum Beispiel eine Duplex-PCR zum Einsatz, bei welcher innerhalb der gleichen Reaktion neben einer DNA-Methylierung von *CTLA4* auch die Gesamt-DNA, das heißt die Gesamtmenge und/oder Gesamtzahl Genomkopien der umgewandelten DNA, bestimmt wurde. Die methylierungsspezifische Amplifikation des *CTLA4* Lokus erfolgte mithilfe von Primern der Sequenzen SEQ ID NO:9 und SEQ ID NO:10. Diese Primer amplifizieren die Sequenz, die durch Bisulfit-Konversion der Sequenz SEQ ID NO:13 entsteht. Im Fall von vollständiger Methylierung hat diese umgewandelte Region im Genom die Sequenz SEQ ID NO:12. Die methylierungsspezifische Detektion erfolgte mit einer Sonde der Sequenz SEQ ID NO:11, welche an 5' den Fluoreszenzfarbstoff 6-FAM und an 3' den Quencher BHQ-1 trug. Für die Bestimmung der Gesamt-DNA wurde ein Lokus im *ACTB* Gen methylierungsunabhängig amplifiziert. Dieser Lokus hat im Genom die Sequenz mit der SEQ ID NO:8 und hat nach der Konversion durch Bisulfit die Sequenz mit der SEQ ID NO:7. Diese Sequenz wurde unter Verwendung der Primer mit den Sequenzen SEQ ID NO:4 und SEQ ID NO:5 amplifiziert. Die sequenzspezifische Detektion des Amplifikats erfolgte mit der Sonde der Sequenz SEQ ID NO:6, welche an 5' den Fluoreszenzfarbstoff Atto 647N und an 3' den Quencher BHQ-2 trug.

Die Echtzeit-PCR wurde in 20 µl PCR Reaktionen in jeweils drei unabhängigen Messungen durchgeführt, wobei sich zum Beispiel die folgende Reaktionszusammensetzung eignete: 35 mM Tris-HCl, pH 8,4, 6 mM MgCl₂, 50 mM KCl, 4% Glycerin, 0,25 mM jedes dNTP (dTTP, dATP, dGTP, dCTP), 2 U FastStart *Taq* DNA-Polymerase (Roche Applied Science, Penzberg, Deutschland), 0,4 µM jedes Primers und 0,2 µM jeder Detektionssonde. Die qPCR wurde zum Beispiel mittels eines AB 7500 Fast Real-Time PCR System (Life Technologies Corporation, Carlsbad, CA, USA) durchgeführt. Ein geeignetes Temperaturprofil umfasste beispielsweise folgende Schritte: 20 min bei 95 °C, gefolgt von 45 Zyklen je 45 s bei 56 °C und 15 s bei 95 °C.

Der Anteil von *CTLA4* Methylierung in der konvertierten DNA wurde mittels der DeltaDelta-CT Methode berechnet und prozentual gegenüber einer Standard-DNA mit 100% Methylierung ausgedrückt. Als Standard-DNA wurde künstlich methylierte DNA (CpGenomeTM Universal Methylated DNA; Merck Millipore, Darmstadt, Germany) verwendet, welche zuvor mit dem innuCONVERT Bisulfite All-In-One Kit entsprechend der Herstellerangaben umgewandelt wurde.

Das Ansprechen auf die Immuntherapie wurde retrospektiv nach den Kriterien zur Bewertung immun-bedingten Ansprechens solider Tumoren (*immune-related Response Evaluation Criteria In Solid Tumors* -irRECIST) beurteilt. Für die Überlebensanalyse wurde der Tod als Endpunkt betrachtet. Die Überlebensdauer wurde als der Zeitraum von der ersten Gabe des Immun-Checkpoint Inhibitors bis zum Todeszeitpunkt definiert. Mit den Überlebensdaten wurde eine Kaplan-Meyer Analyse mit Log-Rank-Test durchgeführt. Hazard Ratios wurden anhand des univariaten Cox proportional Hazard Modells berechnet und DNA-Methylierungswerte von *CTLA4* zur Basis 2 logarithmiert. Vergleiche wurden durch einseitige ANOVA und Bonferroni Post-Hoc Tests ausgeführt. Kategoriale Variablen wurden mit dem Chi-Quadrat-Test (χ²-Test) geprüft. Zur statistischen Analyse wurde SPSS, Version 23.0 verwendet (SPSS Inc., Chicago, IL, USA).

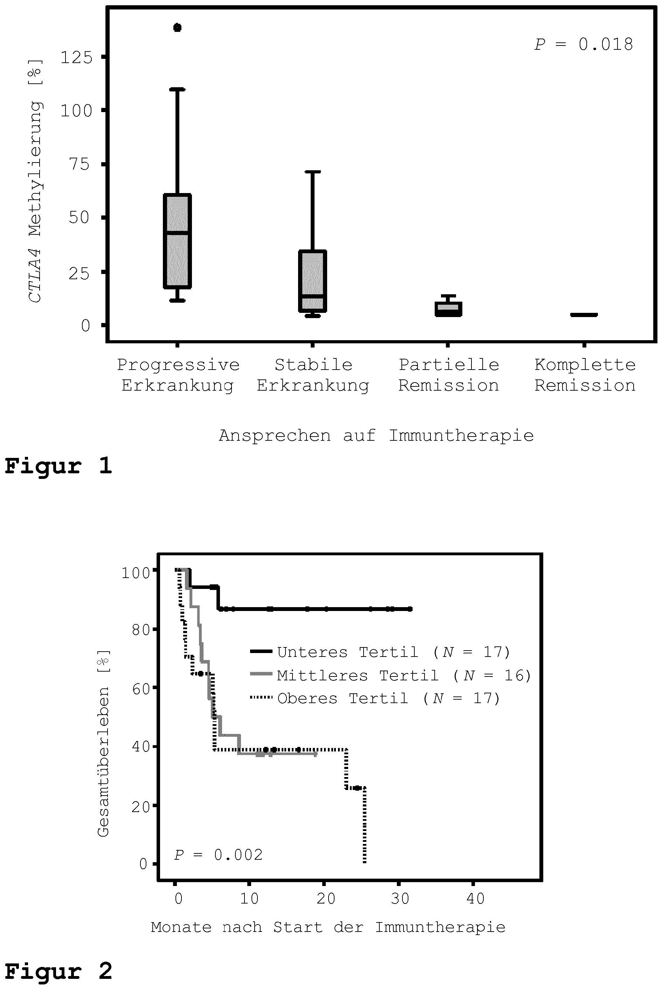

Figur 1 zeigt eine Boxplot-Auswertung des Zusammenhangs zwischen der relativen *CTLA4*-Methylierung (in %, y-Achse) und dem Ansprechen der Patienten, die nach den irRECIST Kriterien gruppiert wurden (x-Achse). Die mittlere *CTLA4*-Methylierung betrug in der Patientengruppe mit fortschreitender Erkrankung 46,1% (95% Konfidenzintervall KI: 31,2-61,0), in der Gruppe mit stabiler Erkrankung 21,6% (95% KI: 11,2-31,9), in der Gruppe mit partieller Remission 7,6% (95% KI: 1,1-14,2) und in der Gruppe mit vollständiger Remission 4,9%. Diese Ergebnisse zeigen deutlich und mit hoher statistischer Signifikanz (p = 0,018), dass eine geringe DNA-Methylierung des *CTLA4* Gens mit einem Ansprechen der malignen Erkrankung auf die Immuntherapie zur Inhibierung des PD-1-Immun-Checkpoint-Signalwegs assoziiert ist. Darüber hinaus zeigen die Ergebnisse, dass auch die Stärke des Ansprechens mit dem Ausmaß der DNA-Methylierung beziehungsweise dem Methylierungsgrad des *CTLA4* Gens korreliert. Demzufolge ist das Ansprechen umso besser, je geringer das *CTLA4* Gen methyliert ist.

Figur 2 zeigt ergänzend eine Kaplan-Meier Analyse des Gesamtüberlebens der 50 Patienten mit malignen Melanomen während der Immuntherapie. Die Patienten wurden entsprechend einer in der pathologischen Klassifizierung gebräuchlichen dreistufigen Bewertung anhand von *CTLA4*-Methylierungstertilen kategorisiert. Die Analyse zeigt dabei ein hochsignifikant (p = 0,002) verlängertes Überleben der Patienten im niedrigsten *CTLA4-*Methylierungstertil im Vergleich zum mittleren und höchsten *CTLA4*-Tertil. Von den 17 Patienten mit der geringsten *CTLA4-*Methylierung im Tumor, die das untere *CTLA4*-Methylierungstertil bilden, lebten 30 Monate nach Beginn der Immuntherapie noch mehr als 80% der Patienten beziehungsweise waren zensiert. Von den 17 Patienten mit der höchsten *CTLA4*-Methylierung im Tumor, die das obere *CTLA4*-Methylierungstertil bilden, sowie den 16 Patienten des mittleren *CTLA4*-Methylierungstertils überlebten weniger als 40% länger als 12 Monaten nach Beginn der Immuntherapie.

Somit konnte der Erfinder erstmals zeigen, dass eine DNA-Methylierungsanalyse des immunregulatorischen Gens *CTLA4* von Zellen einer malignen Erkrankung und/oder von mit den Zellen der malignen Erkrankung wechselwirkenden Immunzellen mit hoher Zuverlässigkeit eine Vorhersage des Ansprechens der malignen Erkrankung auf eine Immuntherapie, welche dazu ausgelegt ist, einen PD-1-Immun-Checkpoint-Signalweg zu inhibieren, ermöglicht. Entsprechend konnte auch gezeigt werden, dass Anwesenheit, Abwesenheit oder Ausmaß einer DNA-Methylierung von *CTLA4* einen zuverlässigen Biomarker zur Vorhersage eines Ansprechens der malignen Erkrankung auf eine solche Immuntherapie darstellt.

### Beispiel 2: Bestimmung der Komethylierung von CTLA4, CD86, CD28, CD80 und ICOS in verschiedenen malignen Erkrankungen

Im Rahmen der vorliegenden Erfindung wurde weiterhin erkannt, dass das Ansprechverhalten eines Patienten auf die Immuntherapie nicht nur anhand der DNA-Methylierungsanalyse von *CTLA4* bestimmt werden kann, sondern auch anhand der DNA-Methylierungsanalyse eines immunregulatorischen Gens, dessen DNA-Methylierung mit der DNA-Methylierung von *CTLA4* korreliert ist. Als besonders geeignet erwiesen sich hierbei die immunregulatorischen Gene *CD28, CD80, CD86* und/oder *ICOS.* Diese Gene stehen in einem funktionalen Zusammenhang mit *CTLA4.* Darüber hinaus sind *CD28* und *ICOS* im Genom auch in unmittelbarer Nähe zu *CTLA4* lokalisiert.

Die Komethylierung von *CTLA4, CD28, CD80, CD86* und *ICOS* wurde mithilfe einer genomweiten die DNA-Methylierungsanalyse untersucht, wofür sich zum Beispiel der Infinium HumanMethylation450 BeadChip (Illumina, Inc., San Diego, CA, USA) entsprechend der Herstellerangaben eignete. Die Generierung der HumanMethylation450 BeadChip Rohdaten erfolgte wie vom TCGA Research Network (http://cancergenome.nih.gov/) beschrieben. Insgesamt wurden Rohdaten von 419 Urothelkarzinomen der Blase, 797 Brustkrebstumoren, 530 Plattenepithelkarzinomen des Kopf-und Hals-Bereichs, 325 klarzelligen Nierenzellkarzinomen, 475 Adenokarzinomen der Lunge, 370 Plattenepithelkarzinomen der Lunge, 265 Sarkomen und 473 kutanen Melanomen verwendet und retrospektiv analysiert.

Die DNA-Methylierungsanalyse umfasste verschiedene Bereiche der Gene, die durch die Beads des HumanMethylation450 BeadChip abgedeckt werden: SEQ ID NO:16 bis SEQ ID NO:21 und SEQ ID NO:37 bis SEQ ID NO:42 erfassen eine DNA-Methylierung unmittelbar vor dem *CD28* Gen. SEQ ID NO:22 bis SEQ ID NO:26 und SEQ ID NO:43 bis SEQ ID NO:47 erfassen eine DNA-Methylierung in dem codierenden Bereich von *CD28.* SEQ ID NO:27 bis SEQ ID NO:29 und SEQ ID NO:48 erfassen eine DNA-Methylierung in einem Bereich zwischen *CD28* und *CTLA4.* SEQ ID NO:30 bis SEQ ID NO:33, SEQ ID NO:49 und SEQ ID NO:50 erfassen eine DNA-Methylierung in dem codierenden Bereich des *CTLA4* Gens. SEQ ID NO:52 erlaubt eine DNA-Methylierungsanalyse eines Bereichs zwischen *CTLA4* und *ICOS.* SEQ ID NO:34 bis SEQ ID NO:36 und SEQ ID NO:53 bis SEQ ID NO:56 erfassen eine DNA-Methylierung eines codierenden Bereichs des *ICOS* Gens. SEQ ID NO:57 bis SEQ ID NO:64 erfassen eine DNA-Methylierung im codierenden Bereich und dem Promotor des *CD80* Gens. SEQ ID NO:65 bis SEQ ID NO:77 erfassen eine DNA-Methylierung im codierenden Bereich und dem Promotor des *CD86* Gens. Aus dem Sequenzprotokoll ist ersichtlich, welche Beads des HumanMethylation450 BeadChips für die DNA-Methylierungsanalyse der jeweiligen Sequenzen herangezogen wurden.

Die Komethylierung dieser Genloci wurde in Bezug auf die DNA-Methylierung der Sequenz SEQ ID NO:51 (Bead cg08460026) des *CTLA4* Gens bestimmt. Diese enthält dieselben vier CpG-Dinukleotide wie SEQ ID NO:13, die in Beispiel 1 mittels quantitativer Echtzeit-PCR untersucht wurden. Dadurch lässt sich ein direkter Bezug zu den in Beispiel 1 dargestellten Ergebnissen zur Vorhersage des Ansprechens der malignen Erkrankung auf die Immuntherapie herstellen.

Zunächst wurde anhand der HumanMethylation450 BeadChip Rohdaten für jedes der betrachteten Bead-Paare und für jede Patientenprobe ein Methylierungswert berechnet. Dazu wurde das Signal des Beads eines Paares, das an die methylierte Variante bindet (S_M), zu dem Signal des Beads des Paares, das an die unmethylierte DNA bindet (S_U), ins Verhältnis gesetzt. Ein Bead umfasst ein gebundenes Oligonukleotid und wird hier auch als Sonde bezeichnet. Die Berechnung der DNA-Methylierung anhand des Verhältnisses erfolgte nach der Gleichung: Methylierung = (Intensität Sonde S_M) / ((Intensität Sonde S_M) + (Intensität Sonde S_U)).

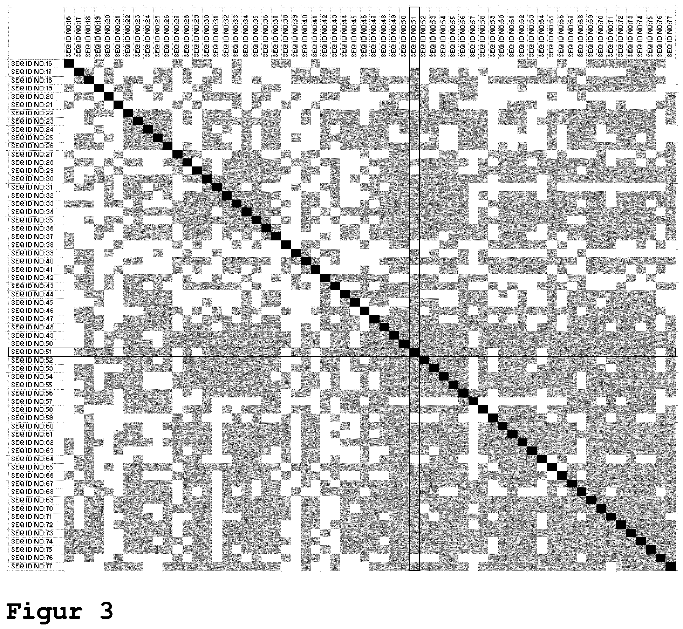

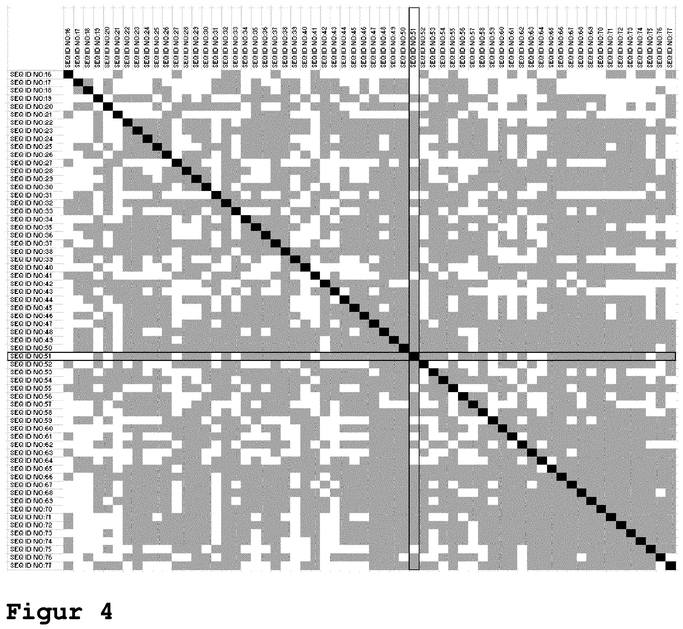

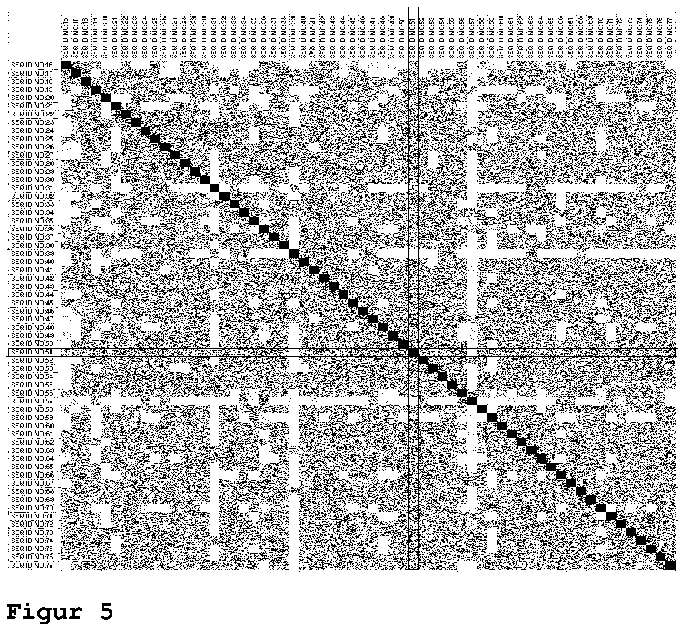

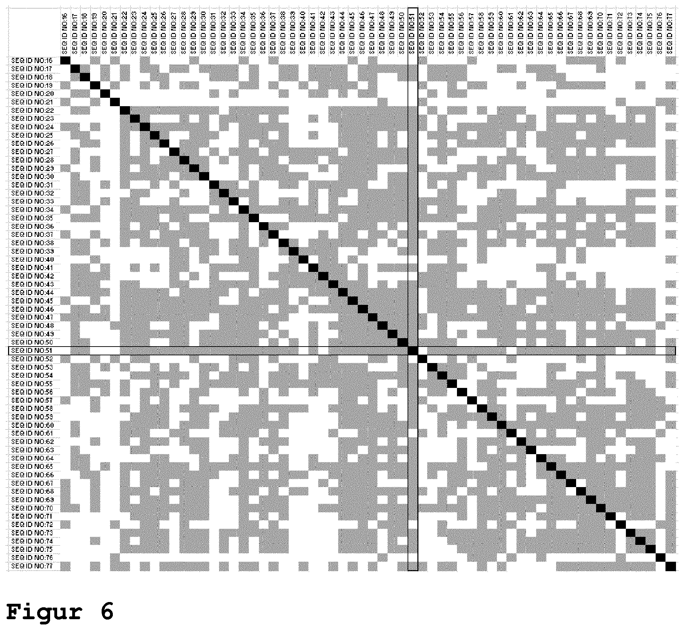

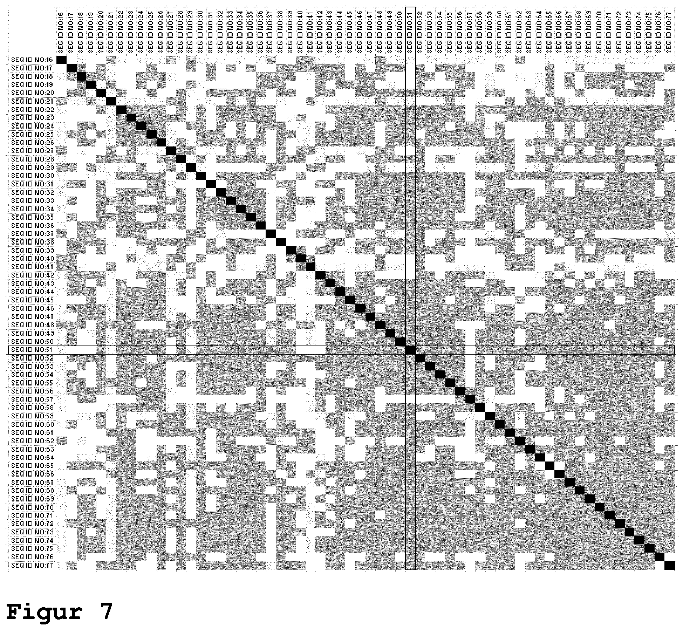

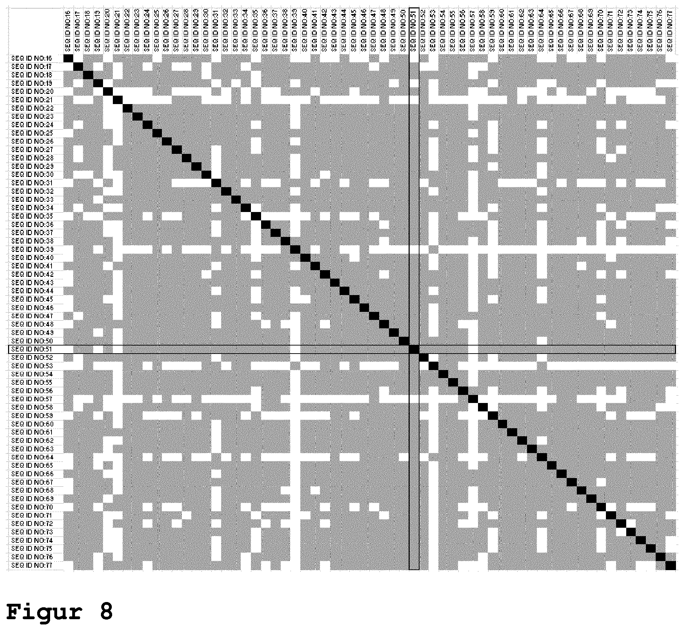

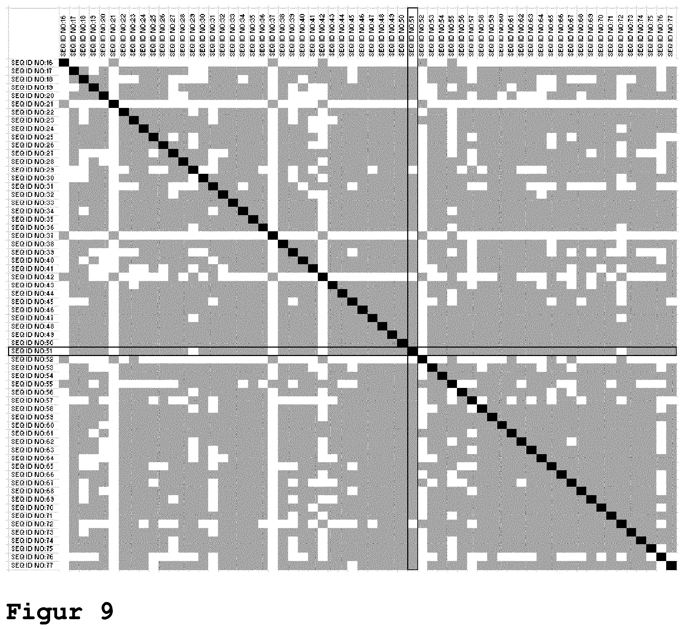

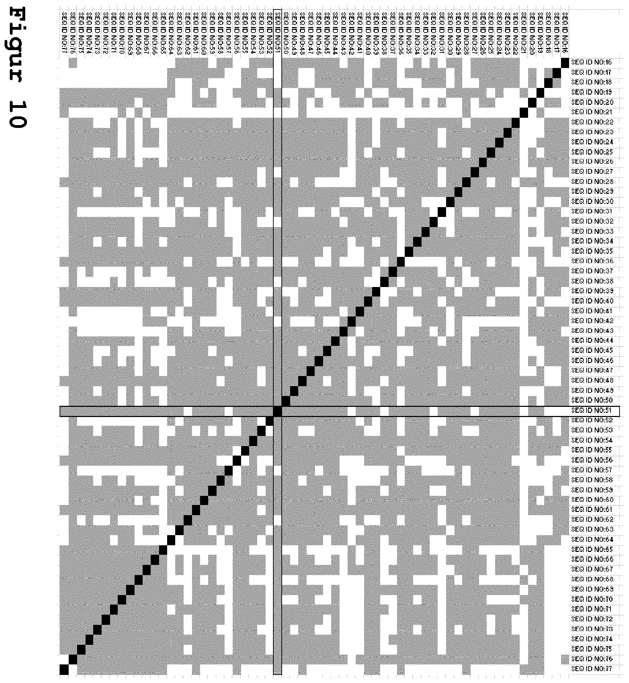

Die Figuren 3 bis 10 zeigen das Ausmaß der Komethylierung der analysierten Genloci in den verschiedenen Typen von malignen Erkrankungen. Die Genloci sind vertikal und horizontal jeweils fortlaufend durch SEQ ID NO:16 (vertikal: oben, horizontal: links) bis SEQ ID NO:77 (vertikal: unten, horizontal: rechts) gekennzeichnet. SEQ ID NO:51 (schwarz umrandet) umfasst SEQ ID NO:13, die in Beispiel 1 mittels quantitativer Echtzeit-PCR zur Erfassung der DNA-Methylierung des *CTLA4* Gens untersucht wurde.

Die Matrizen zeigen, ob die DNA-Methylierung einer bestimmten untersuchten Sequenz mit der DNA-Methylierung der jeweils anderen Sequenzen korreliert ist. Eine statistisch signifikante Korrelation zwischen der DNA-Methylierung von zwei Sequenzen, das heißt ein p-Wert der Spearman Rang-Korrelation von kleiner 0.05, ist in der Matrix als graues Kästchen dargestellt. Weiße Felder zeigen an, dass keine signifikante Korrelation (p ≥ 0,05) der DNA-Methylierung der beiden entsprechenden Sequenzen vorliegt.

Die Ergebnisse zeigen deutlich, dass alle von *CD28, CD80, CD86* und *ICOS* analysierten Genbereiche eine DNA-Methylierung aufweisen, die in den untersuchten malignen Erkrankungen signifikant positiv mit der DNA-Methylierung von *CTLA4* korreliert ist. Aufgrund dieser Komethylierung konnte der Erfinder den Beweis führen, dass analog zu *CTLA4* ebenfalls eine Assoziation zwischen der DNA-Methylierung der Gene *CD28, CD80, CD86* und *ICOS* und dem Ansprechen der malignen Erkrankung auf die Immuntherapie gemäß Beispiel 1 besteht. Folgerichtig ermöglicht - alternativ oder zusätzlich zur DNA-Methylierungsanalyse von *CTLA4* - eine DNA-Methylierungsanalyse von einem oder mehreren der Gene *CD28, CD80, CD86* und *ICOS* die Voraussage des Ansprechverhaltens einer malignen Erkrankung auf eine Immuntherapie, welche dazu ausgelegt ist, einen PD-1-Immun-Checkpoint-Signalweg zu inhibieren.

Aus der dargelegten Komethylierung ergibt sich der weitere besondere Vorteil der Erfindung, dass sich die DNA-Methylierungsanalysen der Gene *CTLA4, CD28, CD80, CD86* und *ICOS* auch gegenseitig funktionell beeinflussen können. Da zum Beispiel, wie oben gezeigt, eine geringe DNA-Methylierung eines erfindungsgemäßen Gens mit einer geringen DNA-Methylierung eines weiteren erfindungsgemäßen Gens korreliert, hat eine kombinierte DNA-Methylierungsanalyse beider Gene die Wirkung, dass sich beide Einzelergebnisse zu einem besonders robusten Gesamtergebnis in Bezug auf die Vorhersage des Ansprechverhaltens der malignen Erkrankung auf die Immuntherapie ergänzen. Dieser gegenseitige "Konsolidierungseffekt" der DNA-Methylierungsanalyse von den erfindungsgemäßen Genen bei der Vorhersage des Ansprechverhaltens ist insbesondere bei kleinen Probenmengen vorteilhaft, bei denen die zur Verfügung stehende DNA-Menge eines einzelnen immunregulatorischen Gens nahe an der unteren Nachweisgrenze einer DNA-Methylierungsanalyse liegt. Dadurch löst die vorliegende Erfindung das Problem, dass im klinischen Alltag häufig nur kleine Gewebe- oder Flüssigbiopsien mit geringen Mengen an DNA von Zellen der malignen Erkrankung und/oder Immunzellen zur Verfügung stehen, anhand derer das Ansprechverhalten der malignen Erkrankung zuverlässig vorhergesagt werden muss.

## Patentansprüche

1. Ein Verfahren zur Vorhersage eines Ansprechens einer malignen Erkrankung auf eine Immuntherapie, welche dazu ausgelegt ist, einen PD-1-Immun-Checkpoint-Signalweg zu inhibieren,
wobei eine DNA-Methylierungsanalyse zumindest eines Teils eines immunregulatorischen Gens ausgewählt aus *CTLA4, CD86, CD28, CD80* und/oder *ICOS* von Zellen der malignen Erkrankung und/oder von mit den Zellen der malignen Erkrankung wechselwirkenden Immunzellen durchgeführt und
anhand von Anwesenheit, Abwesenheit und/oder Ausmaß einer DNA-Methylierung des immunregulatorischen Gens das Ansprechen der malignen Erkrankung auf die Immuntherapie vorhergesagt wird,
wobei die Immuntherapie einen Wirkstoff umfasst, welcher den PD-1-Immun-Checkpoint-Signalweg durch eine Bindung des Wirkstoffs an PD-1, PD-L1 und/oder PD-L2 inhibiert, wobei der Wirkstoff einen anti-PD-1 Antikörper, einen anti-PD-L1 Antikörper und/oder einen anti-PD-L2 Antikörper umfasst.

2. Das Verfahren nach Anspruch 1, wobei die DNA-Methylierungsanalyse zumindest jeweils einen Teil von mindestens zwei der immunregulatorischen Gene umfasst.

3. Das Verfahren nach einem der Ansprüche 1 oder 2, wobei die mit den Zellen der malignen Erkrankung wechselwirkenden Immunzellen ausgewählt sind aus T-Lymphozyten, B-Lymphozyten, antigenpräsentierenden Zellen und/oder natürlichen Killerzellen.

4. Das Verfahren nach einem der Ansprüche 1 bis 3, wobei die maligne Erkrankung ein Melanom, ein Karzinom, insbesondere ein Plattenepithelkarzinom oder ein Adenokarzinom, eine Leukämie, ein Glioblastom ein Sarkom und/oder ein Lymphom umfasst.

5. Das Verfahren nach einem der Ansprüche 1 bis 4, wobei das Ansprechen der malignen Erkrankung auf die Immuntherapie umso wahrscheinlicher ist, je geringer das Ausmaß der DNA-Methylierung des immunregulatorischen Gens ist, insbesondere wobei die Abwesenheit der DNA-Methylierung des immunregulatorischen Gens anzeigt, dass die maligne Erkrankung wahrscheinlich auf die Immuntherapie anspricht.

6. Das Verfahren nach einem der Ansprüche 1 bis 5, wobei die DNA-Methylierungsanalyse Genkopien des immunregulatorischen Gens von mehreren Zellen der malignen Erkrankung und/oder mehreren der mit den Zellen der malignen Erkrankung wechselwirkenden Immunzellen des umfasst und
wobei das Ansprechen der malignen Erkrankung auf die Immuntherapie wahrscheinlich ist, wenn kleiner gleich 40%, kleiner gleich 35%, kleiner gleich 30%, kleiner gleich 25%, kleiner gleich 20%, kleiner gleich 15%, kleiner gleich 10% oder kleiner gleich 5% der Genkopien des immunregulatorischen Gens die DNA-Methylierung aufweisen.

7. Ein Verfahren zur Auswahl eines Patienten, welcher an einer malignen Erkrankung leidet, für eine Immuntherapie, welche dazu ausgelegt ist, einen PD-1-Immun-Checkpoint-Signalweg zu inhibieren, umfassend
A) Bereitstellen von Zellen der malignen Erkrankung und/oder von mit den Zellen der malignen Erkrankung wechselwirkenden Immunzellen des Patienten,
B) Durchführen einer DNA-Methylierungsanalyse zumindest eines Teils eines immunregulatorischen Gens ausgewählt aus *CTLA4, CD86, CD28, CD80* und/oder *ICOS* von den Zellen der malignen Erkrankung und/oder von mit den Zellen der malignen Erkrankung wechselwirkenden Immunzellen aus A),
C) Auswählen des Patienten für die Immuntherapie anhand von Anwesenheit, Abwesenheit und/oder Ausmaß einer in B) ermittelten DNA-Methylierung des immunregulatorischen Gens,
wobei die Immuntherapie einen Wirkstoff umfasst, welcher den PD-1-Immun-Checkpoint-Signalweg durch eine Bindung des Wirkstoffs an PD-1, PD-L1 und/oder PD-L2 inhibiert, wobei der Wirkstoff einen anti-PD-1 Antikörper, einen anti-PD-L1 Antikörper und/oder anti-PD-L2 Antikörper umfasst.

8. Verwendung einer DNA-Methylierungsanalyse zumindest eines Teils eines immunregulatorischen Gens ausgewählt aus *CTLA4, CD86, CD28, CD80* und/oder *ICOS* von Zellen einer malignen Erkrankung und/oder von mit den Zellen der malignen Erkrankung wechselwirkenden Immunzellen
a) zur Vorhersage eines Ansprechens der malignen Erkrankung auf eine Immuntherapie,
b) zur individualisierten Auswahl einer Immuntherapie für die malignen Erkrankung, und/oder
c) zur Auswahl eines Patienten, welcher an der malignen Erkrankung leidet, für eine Immuntherapie,
wobei die Immuntherapie dazu ausgelegt ist, einen PD-1-Immun-Checkpoint-Signalweg zu inhibieren und einen Wirkstoff umfasst, welcher den PD-1-Immun-Checkpoint-Signalweg durch eine Bindung des Wirkstoffs an PD-1, PD-L1 und/oder PD-L2 inhibiert, wobei der Wirkstoff einen anti-PD-1 Antikörper, einen anti-PD-L1 Antikörper und/oder anti-PD-L2 Antikörper umfasst.

9. Verwendung von Anwesenheit, Abwesenheit oder Ausmaß einer DNA-Methylierung von zumindest einem Teil eines immunregulatorischen Gens ausgewählt aus *CTLA4, CD86, CD28, CD80* und/oder *ICOS* von Zellen einer malignen Erkrankung und/oder von mit den Zellen der malignen Erkrankung wechselwirkenden Immunzellen als Biomarker
i) zur Vorhersage eines Ansprechens der malignen Erkrankung auf eine Immuntherapie,
ii) zur individualisierten Auswahl einer Immuntherapie für die malignen Erkrankung, und/oder
iii) zur Auswahl eines Patienten, welcher an der malignen Erkrankung leidet, für eine Immuntherapie,
wobei die Immuntherapie dazu ausgelegt ist, einen PD-1-Immun-Checkpoint-Signalweg zu inhibieren und einen Wirkstoff umfasst, welcher den PD-1-Immun-Checkpoint-Signalweg durch eine Bindung des Wirkstoffs an PD-1, PD-L1 und/oder PD-L2 inhibiert, wobei der Wirkstoff einen anti-PD-1 Antikörper, einen anti-PD-L1 Antikörper und/oder anti-PD-L2 Antikörper umfasst.

10. Die Verwendung nach Anspruch 8 oder 9, wobei die Immuntherapie zusätzlich dazu ausgelegt ist, einen CTLA4-Immun-Checkpoint-Signalweg zu inhibieren, insbesondere wobei die Immuntherapie zusätzlich einen anti-CTLA4 Antikörper, einen anti-CD80 Antikörper, einen anti-CD86 Antikörper und/oder einen anti-CD28 Antikörper umfasst.

11. Verwendung eines Kits zur Durchführung eines Verfahrens nach einem der Ansprüche 1 bis 7, umfassend Reagenzien für die DNA-Methylierungsanalyse zumindest eines Teils eines immunregulatorischen Gens ausgewählt aus *CTLA4, CD86, CD28, CD80* und/oder *ICOS* von Zellen einer malignen Erkrankung und/oder von mit den Zellen der malignen Erkrankung wechselwirkenden Immunzellen, um Anwesenheit, Abwesenheit und/oder Ausmaß der DNA-Methylierung des immunregulatorischen Gens zu bestimmen.

12. Die Verwendung nach Anspruch 11, wobei die DNA-Methylierungsanalyse zumindest jeweils einen Teil von mindestens zwei der immunregulatorischen Gene umfasst.

## Claims

1. A method for predicting a response of a malignant disease to an immunotherapy configured to inhibit a PD-1 immune checkpoint signalling pathway,
wherein a DNA methylation analysis of at least part of an immunoregulatory gene selected from *CTLA4, CD86, CD28, CD80* and/or *ICOS* is carried out on cells of the malignant disease and/or on immune cells interacting with cells of the malignant disease, and
the response of the malignant disease to the immunotherapy is predicted on the basis of the presence, absence and/or level of DNA methylation of the immunoregulatory gene,
wherein the immunotherapy comprises a pharmaceutical compound that inhibits the PD-1 immune checkpoint signalling pathway by binding of the pharmaceutical compound to PD-1, PD-L1 and/or PD-L2, wherein the pharmaceutical compound comprises an anti-PD-1 antibody, an anti-PD-L1 antibody and/or an anti-PD-L2 antibody.

2. The method according to claim 1, wherein the DNA methylation analysis comprises at least part of at least two of the immunoregulatory genes.

3. The method according to any one of claims 1 or 2, wherein the immune cells interacting with cells of the malignant disease are selected from T-lymphocytes, B-lymphocytes, antigen-presenting cells and/or natural killer cells.

4. The method according to any one of claims 1 to 3, wherein the malignant disease comprises a melanoma, a carcinoma, in particular a squamous cell carcinoma or an adenocarcinoma, a leukaemia, a glioblastoma, a sarcoma and/or a lymphoma.

5. The method according to any one of claims 1 to 4, wherein the lower the level of DNA methylation of the immunoregulatory gene, the more likely the malignant disease is to respond to the immunotherapy, in particular wherein the absence of DNA methylation of the immunoregulatory gene indicates that the malignant disease is likely to respond to the immunotherapy.

6. The method according to any one of claims 1 to 5, wherein the DNA methylation analysis comprises gene copies of the immunoregulatory gene of several cells of the malignant disease and/or of several of the immune cells interacting with cells of the malignant disease, and
wherein the malignant disease is likely to respond to the immunotherapy if less than or equal to 40%, less than or equal to 35%, less than or equal to 30%, less than or equal to 25%, less than or equal to 20%, less than or equal to 15%, less than or equal to 10% or less than or equal to 5% of the gene copies of the immunoregulatory gene comprise the DNA methylation.

7. A method for selecting a subject suffering from a malignant disease for an immunotherapy configured to inhibit a PD-1 immune checkpoint signalling pathway, comprising
A) providing cells of the malignant disease and/or immune cells interacting with cells of the malignant disease of the subject,
B) performing a DNA methylation analysis of at least part of an immunoregulatory gene selected from *CTLA4, CD86, CD28, CD80* and/or *ICOS* of the cells of the malignant disease and/or of the immune cells interacting with cells of the malignant disease from A),
C) selecting the subject for the immunotherapy on the basis of the presence, absence and/or level of a DNA methylation of the immunoregulatory gene determined in B),
wherein the immunotherapy comprises a pharmaceutical compound that inhibits the PD-1 immune checkpoint signalling pathway by binding of the pharmaceutical compound to PD-1, PD-L1 and/or PD-L2, wherein the agent comprises an anti-PD-1 antibody, an anti-PD-L1 antibody and/or anti-PD-L2 antibody.

8. Use of a DNA methylation analysis of at least part of an immunoregulatory gene selected from *CTLA4, CD86, CD28, CD80* and/or *ICOS* of cells of a malignant disease and/or of immune cells interacting with cells of the malignant disease
a) for predicting a response of the malignant disease to an immunotherapy,
b) for the individualised selection of an immunotherapy for the malignant disease, and/or
c) for selecting a subject suffering from the malignant disease for an immunotherapy,
wherein the immunotherapy is designed to inhibit a PD-1 immune checkpoint signalling pathway and comprises a pharmaceutical compound which inhibits the PD-1 immune checkpoint signalling pathway by binding of the pharmaceutical compound to PD-1, PD-L1 and/or PD-L2, wherein the pharmaceutical compound comprises an anti-PD-1 antibody, an anti-PD-L1 antibody and/or anti-PD-L2 antibody.

9. Use of the presence, absence or level of DNA methylation of at least part of an immunoregulatory gene selected from *CTLA4, CD86, CD28, CD80* and/or *ICOS* of cells of a malignant disease and/or of immune cells interacting with cells of the malignant disease as a biomarker
i) for predicting a response of the malignant disease to an immunotherapy,
ii) for the individualised selection of an immunotherapy for the malignant disease, and/or
iii) for selecting a subject suffering from the malignant disease for an immunotherapy,
wherein the immunotherapy is configured to inhibit a PD-1 immune checkpoint signalling pathway and comprises a pharmaceutical compound which inhibits the PD-1 immune checkpoint signalling pathway by binding of the pharmaceutical compound to PD-1, PD-L1 and/or PD-L2, wherein the pharmaceutical compound comprises an anti-PD-1 antibody, an anti-PD-L1 antibody and/or anti-PD-L2 antibody.

10. The use according to any one of claims 8 or 9, wherein the immunotherapy is further configured to inhibit a CTLA4 immune checkpoint signalling pathway, in particular wherein the immunotherapy further comprises an anti-CTLA4 antibody, an anti-CD80 antibody, an anti-CD86 antibody and/or an anti-CD28 antibody.

11. Use of a kit for carrying out a method according to any one of claims 1 to 7, comprising reagents for DNA methylation analysis of at least part of an immunoregulatory gene selected from *CTLA4, CD86, CD28, CD80* and/or *ICOS* of cells of a malignant disease and/or of immune cells interacting with cells of the malignant disease, to determine presence, absence and/or level of DNA methylation of the immunoregulatory gene.

12. The use according to claim 11, wherein the DNA methylation analysis comprises at least part of at least two of the immunoregulatory genes.

## Revendications

1. Procédé permettant de prédire la réponse d'une maladie maligne à une immunothérapie conçue pour inhiber une voie de signalisation du point de contrôle immunitaire PD-1,
dans lequel une analyse de méthylation de l'ADN d'au moins une partie d'un gène immunomodulateur choisi parmi CTLA4, CD86, CD28, CD80 et/ou ICOS de cellules de la maladie maligne et/ou de cellules immunitaires interagissant avec les cellules de la maladie maligne est effectuée et
la réponse de la maladie maligne à l'immunothérapie est prédite sur la base de la présence, de l'absence et/ou du degré d'une méthylation de l'ADN du gène immunomodulateur,
dans lequel l'immunothérapie comprend un agent qui inhibe la voie de signalisation du point de contrôle immunitaire PD-1 par liaison de l'agent à PD-1, PD-L1 et/ou PD-L2, dans lequel l'agent comprend un anticorps anti-PD-1, un anticorps anti-PD-L1 et/ou un anticorps anti-PD-L2.

2. Procédé selon la revendication 1, dans lequel l'analyse de méthylation de l'ADN comprend au moins respectivement une partie d'au moins deux des gènes immunomodulateurs.

3. Procédé selon l'une des revendications 1 ou 2, dans lequel les cellules immunitaires interagissant avec les cellules de la maladie maligne sont choisies parmi lymphocytes T, lymphocytes B, cellules présentatrices d'antigènes et/ou cellules tueuses naturelles.

4. Procédé selon l'une des revendications 1 à 3, dans lequel la maladie maligne comprend un mélanome, un carcinome, en particulier un carcinome épidermoïde ou un adénocarcinome, une leucémie, un glioblastome, un sarcome et/ou un lymphome.

5. Procédé selon l'une des revendications 1 à 4, dans lequel la réponse de la maladie maligne à l'immunothérapie est d'autant plus probable que le degré de méthylation de l'ADN du gène immunomodulateur est faible, en particulier dans lequel l'absence de méthylation de l'ADN du gène immunomodulateur indique que la maladie maligne est susceptible de répondre à l'immunothérapie.

6. Procédé selon l'une des revendications 1 à 5, dans lequel l'analyse de méthylation de l'ADN comprend des copies de gène du gène immunomodulateur de plusieurs cellules de la maladie maligne et/ou de plusieurs cellules immunitaires parmi les cellules immunitaires interagissant avec les cellules de la maladie maligne, et
dans lequel la réponse de la maladie maligne à l'immunothérapie est probable si 40 % ou moins, 35 % ou moins, 30 % ou moins, 25 % ou moins, 20 % ou moins, 15 % ou moins, 10 % ou moins ou 5 % ou moins des copies de gène du gène immunomodulateur présentent la méthylation de l'ADN.

7. Procédé pour la sélection d'un patient souffrant d'une maladie maligne pour une immunothérapie conçue pour inhiber une voie de signalisation du point de contrôle immunitaire PD-1, comprenant
A) la fourniture de cellules de la maladie maligne et/ou de cellules immunitaires du patient interagissant avec les cellules de la maladie maligne,
B) la réalisation d'une analyse de méthylation de l'ADN d'au moins une partie d'un gène immunomodulateur choisi parmi CTLA4, CD86, CD28, CD80 et/ou ICOS des cellules de la maladie maligne et/ou de cellules immunitaires de A) interagissant avec les cellules de la maladie maligne,
C) la sélection du patient pour l'immunothérapie sur la base de la présence, de l'absence et/ou du degré d'une méthylation de l'ADN du gène immunomodulateur déterminée en B),
dans lequel l'immunothérapie comprend un agent qui inhibe la voie de signalisation du point de contrôle immunitaire PD-1 par liaison de l'agent à PD-1, PD-L1 et/ou PD-L2, dans lequel l'agent comprend un anticorps anti-PD-1, un anticorps anti-PD-L1 et/ou un anticorps anti-PD-L2.

8. Utilisation d'une analyse de méthylation de l'ADN d'au moins une partie d'un gène immunomodulateur choisi parmi CTLA4, CD86, CD28, CD80 et/ou ICOS de cellules d'une maladie maligne et/ou de cellules immunitaires interagissant avec les cellules de la maladie maligne
a) pour la prédiction de la réponse de la maladie maligne à l'immunothérapie,
b) pour le choix individualisé d'une immunothérapie pour la maladie maligne, et/ou
c) pour la sélection d'un patient souffrant de la maladie maligne pour une immunothérapie,
dans laquelle l'immunothérapie est conçue pour inhiber une voie de signalisation du point de contrôle immunitaire PD-1 et comprend un agent qui inhibe la voie de signalisation du point de contrôle immunitaire PD-1 par liaison de l'agent à PD-1, PD-L1 et/ou PD-L2, dans laquelle l'agent comprend un anticorps anti-PD-1, un anticorps anti-PD-L1 et/ou un anticorps anti-PD-L2.

9. Utilisation de la présence, de l'absence ou du degré d'une méthylation de l'ADN d'au moins une partie d'un gène immunomodulateur choisi parmi CTLA4, CD86, CD28, CD80 et/ou ICOS de cellules d'une maladie maligne et/ou de cellules immunitaires interagissant avec les cellules de la maladie maligne comme biomarqueurs
i) pour la prédiction de la réponse de la maladie maligne à l'immunothérapie,
ii) pour le choix individualisé d'une immunothérapie pour la maladie maligne, et/ou
iii) pour la sélection d'un patient souffrant de la maladie maligne pour une immunothérapie,
dans laquelle l'immunothérapie est conçue pour inhiber une voie de signalisation du point de contrôle immunitaire PD-1 et comprend un agent qui inhibe la voie de signalisation du point de contrôle immunitaire PD-1 par liaison de l'agent à PD-1, PD-L1 et/ou PD-L2, dans laquelle l'agent comprend un anticorps anti-PD-1, un anticorps anti-PD-L1 et/ou un anticorps anti-PD-L2.

10. Utilisation selon la revendication 8 ou 9, dans laquelle l'immunothérapie est en outre conçue pour inhiber une voie de signalisation du point de contrôle immunitaire CTLA4, en particulier dans laquelle l'immunothérapie comprend en outre un anticorps anti-CTLA4, un anticorps anti-CD80, un anticorps anti-CD86 et/ou un anticorps anti-CD28.

11. Utilisation d'un kit pour la mise en œuvre d'un procédé selon l'une des revendications 1 à 7, comprenant des réactifs pour l'analyse de la méthylation de l'ADN d'au moins une partie d'un gène immunomodulateur choisi parmi CTLA4, CD86, CD28, CD80 et/ou ICOS de cellules d'une maladie maligne et/ou de cellules immunitaires interagissant avec les cellules de la maladie maligne, afin de déterminer la présence, l'absence et/ou le degré de méthylation de l'ADN du gène immunomodulateur.

12. Utilisation selon la revendication 11, dans laquelle l'analyse de méthylation de l'ADN comprend au moins respectivement une partie d'au moins deux des gènes immunomodulateurs..
